# EUROPEAN PATENT APPLICATION

(11) **EP 1 603 070 A2**
(43) Date of publication of application: **07.12.2005**
(21) Application number: 05011739.9
(22) Date of filing: 31.05.2005
(51) Int. Cl.: G06F 19/00

(54) **Medical image storage apparatus protecting personal information**

(30) Priority: 01.06.2004 JP 2004162806
(71) Applicant: KABUSHIKI KAISHA TOSHIBA, Tokyo (JP); Toshiba Medical Systems Corporation, Otawara-shi, Tochigi-ken (JP)
(72) Inventor: Fukuda, Megumu c/o Intellectual Property Dept., Otawara-shi Tochigi (JP); Fukunaga, Tomohisa c/o Intellectual Property Dept., Otawara-shi Tochigi (JP)
(74) Representative: Kramer - Barske - Schmidtchen

(57) **Abstract**

A medical image storage apparatus includes a receiving unit, a table preparation unit, a first storage unit, a second storage unit, a protection unit, a recapturing unit and an output unit.

The receiving unit is configured to receive medical image information resulting from a medical imaging apparatus. The table preparation unit is configured to prepare a table of a relationship between predetermined personal information concerning the medical image information and image information prepared by removing the predetermined personal information from the medical image information. The first storage unit is configured to store the predetermined personal information. The second storage unit is configured to store the image information. The protection unit is configured to make the table unavailable, e.g. by encryption or writing to an external memory unit. The recapturing unit is configured to recapture the table. The output unit is configured to read the predetermined personal information from the first storage unit and the image information from the second storage unit based on the recaptured table and to prepare and output the medical image information.

## Description

### CROSS-REFERENCE TO RELATED APPLICATION

This application is based upon and claims the benefit of priority from prior Japanese Patent Application No. P2004-162806, filed on June 1, 2004, the entire contents of which are incorporated herein by reference.

### BACKGROUND OF THE INVENTION

### FIELD OF THE INVENTION

The present invention relates to a medical image storage apparatus for storing medical image information including personal information of a patient. The present invention also relates co a medical imaging apparatus for generating and storing the medical image information. The present invention still relates to a medical image system including a medical image storage apparatus and a medical imaging apparatus. The present invention further relates to a method of scoring the medical image information.

### DISCUSSION OF THE BACKGROUND

Medical image information is generated from medical imaging apparatuses such as an X-ray diagnosis apparatus an X-ray computed tomography apparatus a magnetic resonance imaging apparatus, an ultrasound diagnosis apparatus, a positron emission tomography apparatus, and the like. The medical image information usually includes image data a and accompanying information and is stored in a medical image storage apparatus. The accompanying information usually includes examination information and patient information. Therefore, the medical image information includes personal information such as a patient name, patient identification information, and the like, which can identify a patient of the image data. This means that the medical image information is included in a category of individual privacy information and should be protected according to several acts including the Health Insurance Portability and Accountability Act (HIPPA).

Although the medical image information should be administered strictly and protected from the information leakage, it is not possible to deny that there is a posaibility that personal information included in the medical image information would be leaked when the medical image information is used for the purpose other than a medical practice. For example, it may occur when the medical image information is used to research images based on the medical image information or when the medical image information is used during a maintenance service of the medical image storage apparatus.

In order to solve this type of problem, for example, Japanese Patent Application Publication No. 2002-197186 describes a technique of storing information disclosure procedures set by a patient or his/her doctor in a medical information management system in advance and allowing to refer to medical image information only in accordance with the procedures so that the medical image information could not be unintentionally disclosed.

This technique, however, still allows the doctor to edit or process the medical image information irrespective of the purpose of use as long as the doctor is allowed to access the medical image information. Therefore, when the medical image information is used for the purpose of researching images based on the medical image information or for other non-medical purpose, it may still occur that part of personal information included in the medical image information is leaked by careless operations since it is cumbersome to remove the personal information from the medical image information.

Also when a maintenance service staff maintains the medical image storage apparatus, the staff may happen to leak part of the personal information by mistake if the staff is not so familiar with treating the personal information.

Meanwhile, there is no way for the patient to see if the medical image information is strictly archived in security and used in an appropriate manner.

As a technique of securing personal information. Japanese Patent Application Publication Nos. 2000-13614 and 2002-247026 describe a technique of dividing the medical image information into image data and accompanying information and encrypting the accompanying information when the medical image information is transmitted through a network so as to avoid personal information included in the accompanying information from being unintentionally taken by the third person during the transmission. This is, however, only for the security during the transmission and the encrypted accompanying information is decrypted after the reception of the medical image information. These documents neither describe nor suggest any good idea for securing the personal information and solving the above-mentioned problems once after the medical image information has been stored in the medical image storage apparatus or the like.

### SUMMARY OF THE INVENTION

According to the first aspect of the present invention, there is provided a medical image storage apparatus. The apparatus includes a receiving unit, a cable preparation unit, the first storage unit, the second storage unit, the first unit, the second unit, and an output unit. The receiving unit is configured to receive medical image information resulting from a medical imaging apparatus The cable preparation unit is configured to prepare a table of a relationship between predetermined personal information concerning the medical image information and image information prepared by removing the predetermined personal information from the medical image information. The first storage unit is configured to store the predetermined personal information. The second storage unit is configured to store the image information. The first unit is configured to make the table unavailable. The second unit is configured to recapture the table. The output unit is configured to read the predetermined personal information from the first storage unit and the image information from the second storage unit based on the recaptured table and to prepare and output the medical image information.

According to the second aspect of the present invention, there is provided a medical imaging apparatus, The apparatus includes an imaging unit, a table preparation unit, the first storage unit, the second storage unit, the first unit, the second unit, and an output unit. The imaging unit is configured to generate medical image information. The cable preparation unit is configured to prepare a table of a relationship between predetermined personal information concerning the medical image information and image information based on the medical image information. The first storage unit is configured to store the predetermined personal information. The second storage unit is configured to store the image information. The first unit is configured to make the table unavailable. The second unit is configured to recapture the table. The output unit is configured to read the predetermined personal information from the first storage unit and the image information from the second storage unit based on the recaptured table and co prepare and output the medical image information.

According to the third aspect of the present invention, there is provided a medical image system including a medical imaging apparatus and a medical image storage apparatus. The system includes an imaging unit, a table preparation unit, the first storage unit, the second storage unit, the first unit, the second unit, and an output unit. The imaging unit is configured to generate medical image information. The table preparation unit is configured to prepare a table of a relationship between predetermined personal information concerning the medical image information and image information based on the medical image information. The first storage unit is configured to store the predetermined personal information. The second storage unit is configured to store the image information. The first unit is configured co make the table unavailable. The second unit is configured to recapture the cable. The output unit is configured to read the predetermined personal information from the first storage unit and the image information from the second storage unit based on the recaptured table and to prepare and output the medical image information.

According co the fourth aspect of the present invention, there is provided a method of storing medical image information. The method begins by receiving medical image information resulting from a medical imaging apparatus. The method continues by preparing a table of a relationship between predetermined personal information concerning the medical image information and image information prepared by removing the predetermined personal information from the medical image information. The method still continues by storing the predetermined personal information. storing the image information, and making the table unavailable.

According to the fifth aspect of the present invention, there is provided a method of storing medical image information. The method begins by generating medical image information. The method continues by preparing a cable of a relationship between predetermined personal informacion concerning the medical image information and image information based on the medical image information. The method still continues by storing the predetermined personal information, storing the image information, and making the table unavailable.

### BRIEF DESCRIPTION OF THE DRAWINGS

A more complete appreciation of embodiments of the present invention and many of its attendant advantages will be readily obtained by reference to the following detailed description considered in connection with the accompanying drawings, in which:
FIG. 1 is a block diagram showing an exemplary configuration of a medical image storage apparatus according to the first embodiment;
PIG. 2 is an illustration showing an example of divining medical image information according to the first embodiment;
FIGS. 3A and 3B are illustrations showing an example of a table to be encrypted according to the first embodiment;
FIG. 4 is a flowchart showing an exemplary flow of scoring the medical image information in the medical image storage apparatus according to the first embodiment:
FIG. 5 is a flowchart showing the first exemplary flow of delaying a transfer or a divining operation according to the first embodiment;
FIG. 6 is a flowchart showing the second exemplary flow of delaying the transfer or the divining operation according to the first embodiment:
FIG. 7 is a flowchart showing the third exemplary flow of delaying the transfer or the divining operation according to the first embodiment:
FIG. 8 is an illustration showing an example of a step co replace one of steps shown in FIG. 5, 6, or 7 ;
FIG. 9 is an illustration showing another example of a step co replace the one step shown in FIG. 5, 6, or 7;
FIG. 10 is a flowchart showing an exemplary flow of outputting the medical image information in the medical image storage apparatus according to the first embodiment;
FIG. 11 is a flowchart showing an exemplary flow of outputting the image information in the medical image storage apparatus according to the first embodiment;
FIG. 12 is a block diagram showing another exemplary configuration of the medical image storage apparatus according co the first embodiment;
FIG. 13 is a block diagram showing an exemplary configuration of a medical image storage apparatus according to the second embodiment;
FIG. 14 is a flowchart showing an exemplary flow of writing the table to an IC card according to the second embodiment;
FIG. 15 is an illustration showing an example of a table to be stored in the IC card according to the second embodiment;
FIG. 16 is a flowchart showing an exemplary flow of outputting the medical image information according to the second embodiment;
FIG. 17 is a block diagram showing a modified exemplary configuration of the medical image storage apparatus according to the second embodiment;
FIG. 18 is a block diagram showing another exemplary configuration of the medical image storage apparatus according to the second embodiment;
FIG. 19 is a block diagram showing an exemplary configuration of a medical image storage apparatus according to the third embodiment;
FIG. 20 is an illustration showing an example of a table to be encrypted according co the third embodiment;
FIG. 21 is a flowchart showing an exemplary flow of outputting the medical image information in the medical image storage apparatus according to the third embodiment;
FIG. 22 is a block diagram showing another exemplary configuration of the medical image storage apparatus according to the third embodiment:
FIG. 23 is a block diagram showing an exemplary configuration of a medical image storage apparatus according to the fourth embodiment;
FIG. 24 is a flowchart showing an exemplary flow of storing the medical image information in the medical image storage apparatus according to the fourth embodiment;
FIG. 25 is a block diagram showing an exemplary configuration of a medical image storage apparatus according co the fifth embodiment;
FIG. 26 is a flowchart showing an exemplary flow of storing the medical image information in the medical image storage apparatus according co the fifth embodiment;
PIG. 27 is a flowchart showing an exemplary flow of outputting the medical image information in the medical image storage apparatus according to the fifth embodiment;
PIG. 28 is a block diagram showing an exemplary configuration of a medical imaging apparatus according co the sixth embodiment;
FIG. 29 is a block diagram showing a modified exemplary configuration of the medical imaging apparatus according to the sixth embodiment;
FIG. 30 is an illustration showing an example of a medical image system according to the seventh embodiment;
FIG. 31 is an illustration showing another example of the medical image system according co the seventh embodiment; and
FIG. 32 is an illustration showing an example of the IC card according to the embodiments.

### DESCRIPTION OF THE PREFERRED EMBODIMENTS

Embodiments of the present invention will be described with reference to the accompanying drawings.

### (First Embodiment)

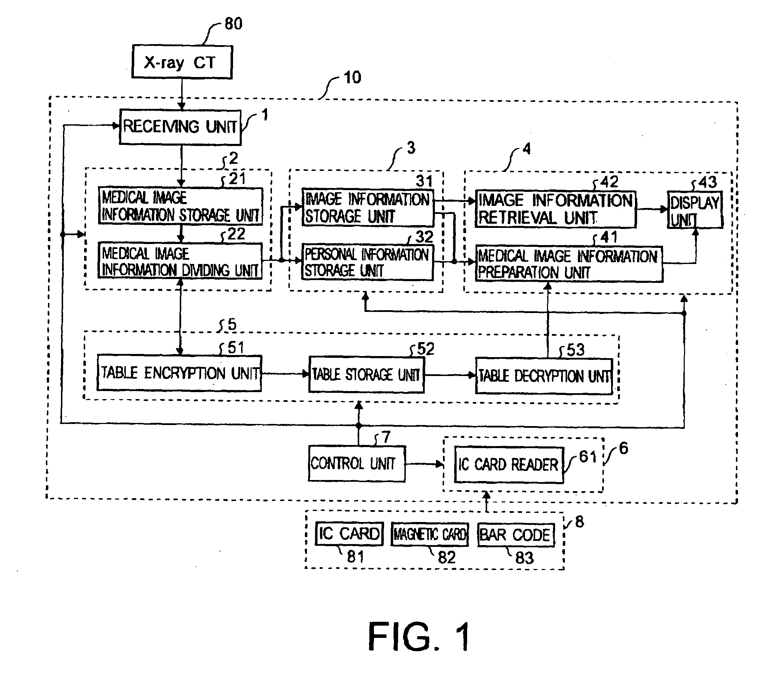

FIG. 1 is a block diagram showing an exemplary configuration of a medical image storage apparatus according to the first embodiment. A medical image storage apparatus 10 such as, for example, an image filing server, may include a receiving unit 1, a medical image information processing unit (hereinafter referred to as an image processing unit) 2, a storage unit 3, a medical image information output unit (hereinafter referred to as an output unit) 4, a table processing unit 5, an operation unit 6, and a control unit 7.

The receiving unit 1 is connected to a medical imaging apparatus such as, for example, an X-ray diagnosis apparatus, an X-ray computed tomography apparatus (hereinafter referred to as an X-ray CT apparatus), a magnetic resonance imaging apparatus, a nuclear medicine diagnosis apparatus, an ultrasound diagnosis apparatus, an endoscope apparatus, and/or the like. In FIG. 1, an X-ray CT apparatus 80 is connected to the receiving unit 1 through a network such as a hospital LAN (Local Area Network). The receiving unit 1 receives medical image information including image data and its accompanying information. The image data pertains to X-ray CT image data generated by the X-ray CT apparatus 80. The image data may also be X-ray CT image data image-processed by the X-ray CT apparatus 80. The image data may be two-dimensional or three-dimensional image data. The image data may include imaging condition information such as for example, imaging parameters and imaging time. The accompanying information includes, for example, patient information and examination information, which concerns the image data. The patient information includes, for example, a patient name, patient identification information, patient's date of birth, patient's sex, and the like, which can usually be treated as personal information of the patient The examination information may include, for example, an examination date, an imaged part of the patient, a type of a medical imaging apparatus (herein, an X-ray CT apparatus), and the like. The image data and the examination information may also include personal information of the patient. The personal information may be information which can identify the patient by itself or with other information of the patient.

The image processing unit 2 may include a medical image information storage unit 21 and a medical image information dividing unit 22. The image processing unit 2 may correspond to 'a table preparation unit'.

The medical image information storage unit 21 temporarily stores the medical image information received by the receiving unit 1. The stored medical image information, which is original medical image information, may be deleted from the medical image information storage unit 21 in accordance with a predetermined condition so that a doctor, a radiological technologist, or the like a (hereinafter referred to as a user) cannot obtain the original medical image information unless permitted or without the patient's help.

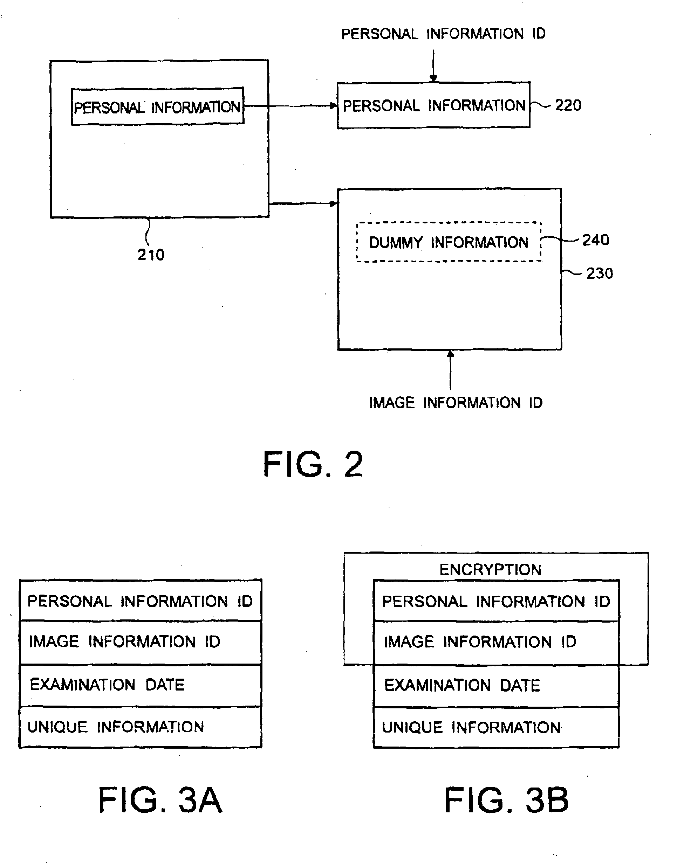

As shown in FIG. 2, the medical image information dividing unit 22 divides the medical image information 210 into predetermined personal information 220 and image information 230. All or part of the personal information may be determined as the predetermined personal information 220 in accordance with a predetermined rule. The predetermined personal information 220 may be extracced from the medical image information 210. The medical image information 210 from which the predetermined personal information 220 is removed is treated as the image information 230. The image information 230 may, however, include dummy information 240 instead of the removed predetermined personalinformation 220. Since a medical imagestorage apparatus can typically recognize where each kind of information is stored, the medical image information dividing unit 22 can extract the predetermined personal information. Especially, when the medical image information is stored in compliance with the DICOM (Digital Imaging and Communication in Medicine) standard, the medical image information dividing unit 22 can extract the predetermined personal information according to tag information.

The medical image informacion dividing unit 22 also issues identification information of the predetermined personal information (hereinafter referred to as personal information ID) and identification information of the image information (hereinafter referred to as image information ID). The personal information ID is given to the predetermined personal information and the predetermined personal information given the personal information ID is transferred to the storage unit 3 as ID-given predetermined personal information. Similarly, the image information ID is given to the image information and the image information given the image information ID is transferred to the storage unit 3 as ID-given image information. The personal information ID is uniquely issued co each medical image information so that the predetermined personal information can be distinguished among the examinations. Similarly, the image information ID is uniquely issued to each medical image information so that the image information can be distinguished among the examinations. Each of the personal information ID and the image information ID does not relate to an issue system in a table storage unit 52 and comprises arbitrary numeral and/or character information which is not displayed in a display unit 43.

The medical image information dividing unit 22 further prepares a table of a relationship between the predetermined personal information and the image information. For example, the medical image information dividing unit 22 prepares a preliminary table including the personal information ID, the image information ID, and the examination date. The examination dace may be obtained from the examination information included in the medical image information. As shown in PIG. 3A, the medical image information dividing unit 22 then prepares the table by adding unique information to the preliminary table. The unique information is uniquely given or issued to each patient such as, for example, patient's health insurance card information, patient's registration card information, password information determined by a patient, or any patient-distinguishable information. This unique information may be input from the operation unit 6. The prepared table is transferred to the table processing unit 5.

The storage unit 3 may be configured by a non-volatile memory device such as, for example, a magnetic disk and include an image information storage unit 31 and a personal information storage unit 32. The image information storage unit 31 stores the ID-given image information. The personal information storage unit 32 stores the ID-given predetermined personal information. The image information storage unit 31 may correspond to 'a second storage unit'. The personal information storage unit 32 may correspond to 'a first storage unit'.

The output unit 4 may include a medical image information preparation unit 41, an image information retrieval unit 42, and the display unit 43. The medical image information preparation unit 41 receives the table (decrypted table) from a cable decryption unit 53 and retrieves from the image information storage unit 31 the ID-given image information whose ID corresponds to the image information ID obtained from the table. The medical image information preparation unit 41 also retrieves from the personal information storage unit 32 the ID-given predetermined personal information whose ID corresponds to the personal information ID obtained from the table. The medical image information preparation unit 41 also prepares medical image information by combining the retrieved image information and the predetermined personal information and transfers the medical image information to the display unit 43. The image information retrieval unit 42 retrieves the (ID-given) image information from the image information storage unit 31 in accordance with a request from the operation unit 6 and transfers the image information to the display unit 43. The display unit 43 displays the medical image informacion when the medical image information is transferred from the medical image information preparation unit 41. In this case, the display unit 43 displays the image information and the personal information included in the medical image information. The display unit 43 displays the image information when the image information is transferred from the image information retrieval unit 42. In this case, the display unit 43 does not display the personal information.

The cable processing unit 5 may include a table encryption unit 51, the table storage unit 52, and a table decryption unit 53. The table encryption unit 51 receives the table transferred from the medical image information dividing unit 22 and encrypts the table. The encrypted cable is transferred to and stored in the cable storage unit 52. The table encryption unit 51 may score an encryption key, particularly when the encryption key is a public key of the patient. The encryption key may alternatively be stored in an IC (Integrated Circuit) card 81, which may be kept and administered by the patient. In this case, the encryption key is read from the IC card 81 through the operation unit 6 and provided to the table encryption unit 51 through the control unit 7. When the table is encrypted only the personal information ID and the image information ID may be encrypted so that the examination date and the unique information can be used in the retrieval of the encrypted cable, as shown in FIG. 3B. The cable encryption unit 51 may correspond co 'a first unit configured to make the table unavailable'.

The table decryption unit 53 retrieves the encrypted table in accordance with the unique information (and the examination date) and decrypts the encrypted table. The decrypted table is transferred to the medical image information preparation unit 41 as the table. In the decryption, the table decryption unit 53 uses a decryption key. When the table encryption unit 51 uses the public key, the table decryption unit 53 uses a private key corresponding to the public key as the decryption key. The decryption key including the private key may be stored in the IC card 81. Therefore, in this case, the decryption key including the private key is read from the IC card 81 through the operation unit 6 and provided to the cable decryption unit 53 through the control unit 7. The table decryption unit 53 may correspond to 'a second unit configured co recapture the table'.

The operation unit 6 may include an IC card reader 61, various switches, and an input device such as, for example, a keyboard, a trackball, a mouse, a magnetic card reader, a bar code reader, and/or the like. When an external memory medium 8 is the IC card 81, the IC card reader 61 is used. When the external memory medium 8 is a magnetic card 82 or a bar code medium 83, the magnetic card reader or the bar code reader may be used. In the following description, embodiments will be explained taking an example of the time when the IC card 81 is used as an example of the external memory medium 8. The external medium 8 may be portable and usually kept and administered by the patient. The operation unit 6 may be operative as an interactive interface. The operation unit 6 may be used to input the unique information, various types of information and instruccions, the encryption key, the decryption key and the like. The unique information, the encryption key and the decryption key may be input through the IC card reader 61.

The control unit 7 may include a CPU (Central Processing Unit) and memory circuitry. The memory circuitry may temporarily score the information, the instructions, the unique information, the encryption key, and the decryption key when these are input from the operation unit 6. These information may be transferred to one or more appropriate units provided in the medical image storage apparatus 10 or used to control one or more appropriate units or over the medical image storage apparatus 10.

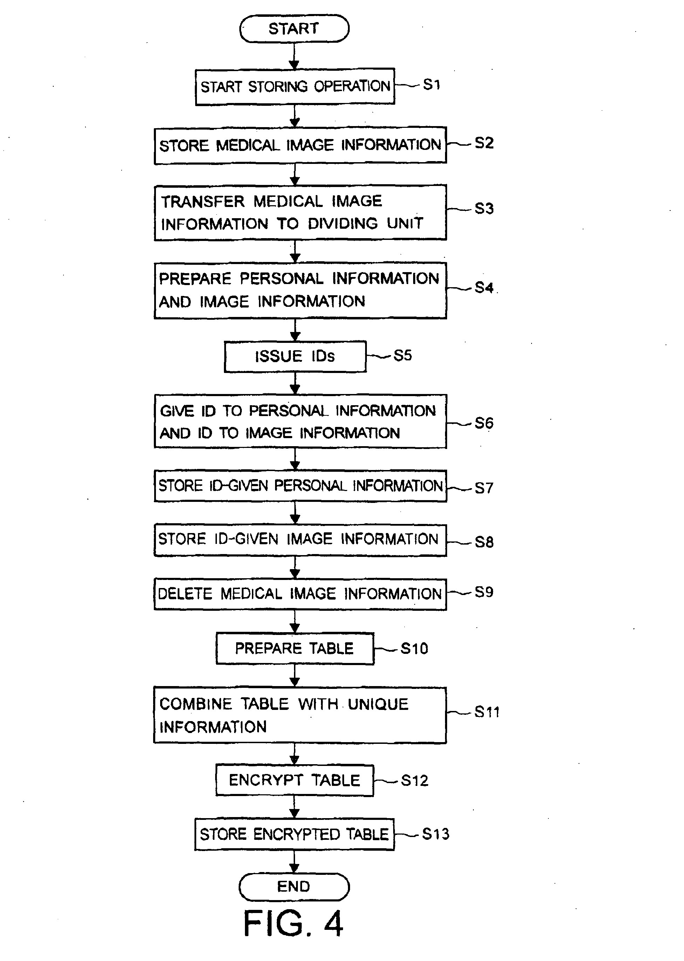

Storing operations of the medical image information will be described with reference co a flowchart shown in FIG. 4. FIG. 4 is a flowchart showing an exemplary flow of storing the medical image information in the medical image storage apparatus 10 according co the first embodiment.

When the x-ray CT apparatus 80 has generated CT image data and its accompanying information as the medical image information, the medical image information is transmitted to the medical image storage apparatus 10 through the hospital LAN. The transmitted medical image information is received by the receiving unit 1. Although the storing operation can be started in response co a user's instruction from the operation unit 6, the storing operation is typically started in accordance with the reception of the medical image information as long as the medical image storage apparatus 10 is ready for receiving and storing the medical image information (step S1).

The received medical image information is temporarily stored in the medical image information storage unit 21 (step S2). The medical image information is then transferred to the medical image information divining unit 22 (step S3) and divided into predetermined personal information and image information. That is, the predetermined personal information is prepared by extracting the predetermined personal information from the medical image information. Also using dummy information, the image information is prepared (step S4).

The transfer of the medical image information from the medical image information storage unit 21 to the medical image information dividing unit 22 means proceeding to the encryption operation. Once the encryption has been conducted, the user cannot obtain the medical image information unless the decryption key which may be administered by the patient is obtained. Since the user usually needs to interpret or diagnose images resulting from the medical image information, identifying the images concerning a specific patient the transfer may not be conducted so soon. Or even if transferred, the dividing operation may not be conducted immediately.

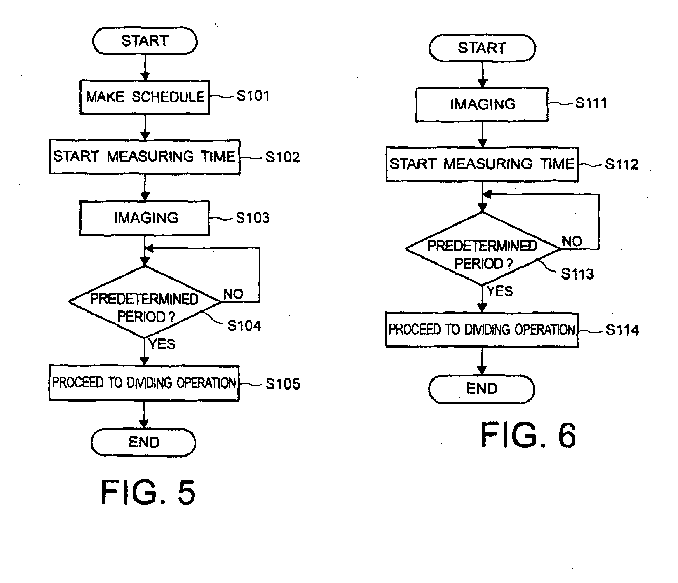

FIG. 5 is a flowchart showing the first exemplary flow of delaying the transfer or the divining operation according to the first embodiment. In advance of imaging operation in the X-ray CT apparatus 80, the user usually makes a schedule of imaging (step S101). When the scheduling has been completed, the control unit 7 starts measuring time (step S102). For example, the completion of the scheduling may be informed to the medical image storage apparatus 10 from an apparatus in which the scheduling has been conducted. In accordance to the schedule, the imaging operation is conducted (step S103). After the imaging, it is determined whether a predetermined time has passed from the start of the time measurement (step S104). Since it is necessary to give the user enough time to interpret images, the predetermined time may be set to be long enough for the user to conduct the interpretation after the imaging. When the control unit 7 has determined that the predetermined time has passed, the control unit 7 controls the image processing unit 2 so that the transfer or the dividing operation is made (step S105).

Time measurement co proceed to the transfer or the dividing operation may alternatively be made as shown in FIG. 6. FIG. 6 is a flowchart showing the second exemplary flow of delaying the transfer or the divining operation according to the first embodiment. The time measurement may start (step S112) when the imaging has been completed (step S111). For example, the control unit 7 may start the measurement in response to the reception of the medical image information by the receiving unit 1. When the control unit 7 has determined that the predetermined time has passed (step S113), the control unit 7 controls the image processing unit 2 so that the transfer or the dividing operation is made (step S114).

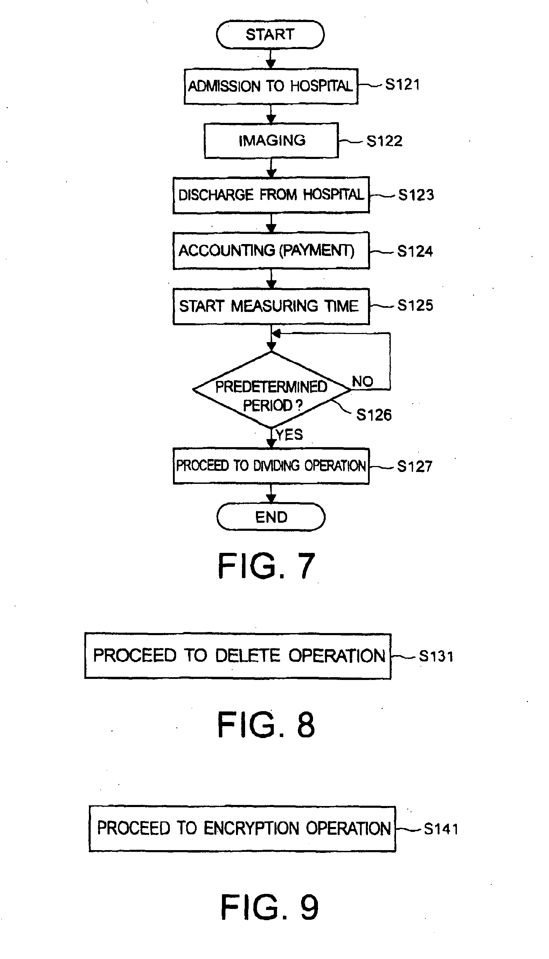

A further alternative way of the measurement is shown in FIG. 7. FIG. 7 is a flowchart showing the third exemplary flow of delaying the transfer or the divining operation according to the first embodiment. This is a case that the time measurement may be started in response co accounting or payment at a hospital. One example of this case may be when the patient is admitted to a hospital. After the patient has been admitted to the hospital (step S121), imaging operation is conducted on the patient (step S122). When the patient is discharged from the hospital (step S123), the patient usually has co make a payment. The payment information may be input to an accounting system by an accounting staff (step S124). For example the completion of the accounting processing may be informed to the medical image storage apparatus 10 from the accounting system. In response to the information from the accounting system, the control unit 7 starts measuring time (step S125). When the control unit 7 has determined that the predetermined time has passed (step S126), the control unit 7 controls the image processing unit 2 so that the transfer or the dividing operation is made (step S127).

According co these examples, the user could have enough time to interpret images based on the medical image information.

Returning co FIG. 4, after the divining operation and preparation of the predetermined personal information and the image information in step S4, the medical image information divining unit 22 issues personal information ID for the predetermined personal information and image information ID for the image information (step S5). The medical image information dividing unit 22 then gives the personal information ID to the predetermined personal information and prepares the ID-given predetermined personal information. Also, the medical image information dividing unit 22 then gives the image information ID to the image information and prepares the ID-given image information (step S6). The ID-given predetermined personal information is stored in the personal information storage unit 32 (step S7). The ID·given image information is stored in the image information storage unit 31 (step S8). After the storage in steps S7 and S8, the medical image information is deleted from the medical image information storage unit 21 so that the user cannot obtain the medical image information without the decryption key (step S9).

For the user's image interpretation work, the time measurement examples have been described with reference to FIGS. 5 to 7. Instead of applying the time measurement examples to the determination of whether to proceed to the dividing operation, similar examples may be applicable to the above delete operation in step S9. For example, step S105 in FIG. 5. S114 in FIG. 6, or S127 in FIG. 7 may be replaced by step S131 shown in FIG. 8. That is, the delete operation in step S9 may be conducted only after an elapse of the predetermined time by the time measurement.

The medical image information dividing unit 22 also prepares a table of a relationship between the predetermined personal intormation and the image information. First, a preliminary cable is prepared, which includes the personal information ID, the image information ID, and an examination date (step S10). Next, the medical image information dividing unit 22 prepares the table by combining the unique information to the preliminary table as shown in FIG. 3A (step S11).

The table is transferred to and encrypted by the table encryption unit 51 (step S12). As described before, the table encryption unit 51 may use the decryption key received from the IC card 81 through the IC card reader 61 or the public key scored in the table encryption unit 51 or other unit in the medical image storage unit 10. When the encryption key is not stored as the public key, an input of the encryption key may be required through a requirement display in the display unit 43. In response to the requirement display, the user may insert the IC card 81 into the IC card reader 61. To be precise, the user may ask the patient to insert the IC card 81 into the IC card reader 61. Even if the public key is not used as the encryption key, the table may be encrypted, by reading the encryption key from the IC card 81 in advance. As shown in FIG. 3B, only the personal information ID and the image information ID may be encrypted in the table encryption unit 51. The time measurement examples for the user's image interpretation may still alternatively be applied to the encryption operation in step S12. For example, step S105 in FIG. 5, S114 in FIG. 6, or S127 in FIG. 7 may be replaced by step S141 shown in FIG. 9. That is, the encryption operation in step S12 may be conducted only after an elapse of the predetermined time by the time measurement. The encrypted table is stored in the table storage unit 52. The storing operation may be completed by this storage.

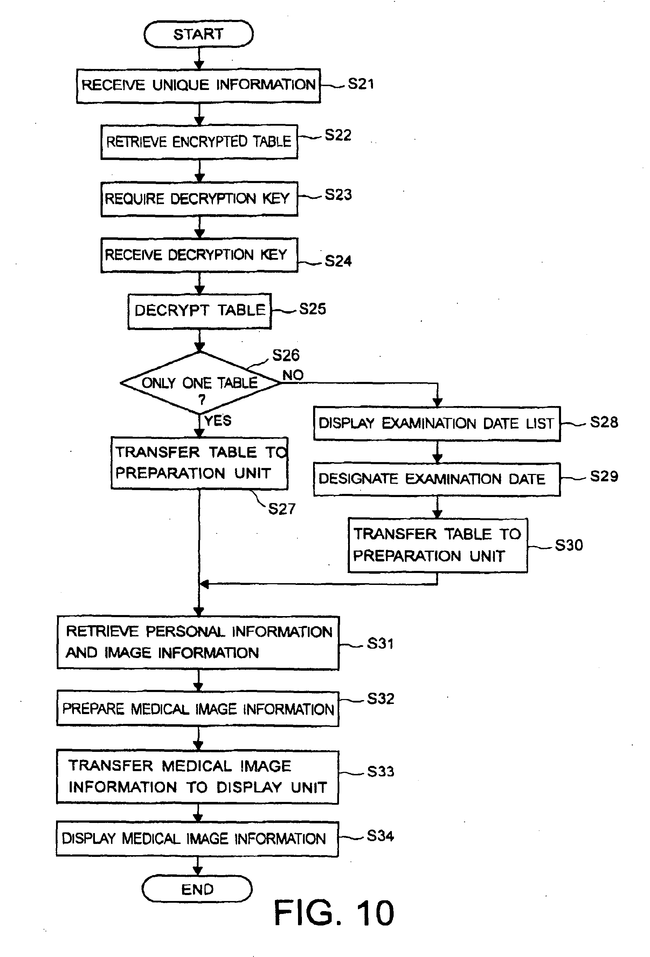

Outputting operations of the medical image information will be described with reference to a flowchart shown in PIG. 10. FIG. 10 is a flowchart showing an examplary flow of outputting the medical image information in the medical image storage apparatus 10 according to the first embodiment. Since the medical image information has been deleted from the medical image storage apparatus 10, the user needs to decrypt the encrypted cable so that the medical image information is reproduced based on the table if the user needs to obtain the medical image information.

The user may input an instruction to start obtaining the medical image information through the operation unit 6. In order to obtain and output the medical image information except for the time of the image interpretation which may have been completed before the encryption, the user then needs to ask the patient to insert the IC card 81 into the IC card reader 61. The user may first input unique information of the patient from the operation unit 6. Or when the unique information is stored in the IC card 61, the user may ask the patient to insert the IC card 81 into the IC card reader 61 so that the unique information is read from the IC card 81 (step S21). The unique information through the operation unit 6 is transferred to the table decryption unit 53. The table decryption unit 53 then compares the transferred unique information to unique information included in the encrypted cable scored in the table storage unit 52 since the unique information included in the cable is not encrypted as shown in FIG. 3B. The table decryption unit 53 retrieves one or more encrypted tables including unique information identical to or corresponding to the transferred unique information (step S22). After the retrieval, an input of the decryption key is required through the requirement display in the display unit 43 (step S23).

In response to the insertion of the IC card 81 into the IC card reader 61, the IC card reader 61 reads the decryption key from the IC card 81 and the table decryption unit 53 receives the decryption key (step S24). As described before, the decryption key may be the private key of the patient. The table decryption unit 53 then decrypts the one or more encrypted tables with the decryption key and obtains one or more tables (step S25). When there is only one cable obtained in step S25 (step S26), the table is transferred to the medical image information preparation unit 41 (step S27). When there are two or more tables obtained in step S25, the examination dates included in the respective tables are displayed as a list of examination dates in the display unit 43 (step S28). When the user designates one of the listed examination dates, using the operation unit 6 (step S29), the table corresponding to the designated examination date is transferred to the medical image information preparation unit 41 (step S30).

The medical image information preparation unit 41 retrieves the ID-given predetermined personal information scored in the personal information storage unit 32 in accordance with the personal information ID included in the transferred table and the ID-given image information scored in the image information storage unit 32 in accordance with the image information ID included in the transferred table (step S31). The medical image information preparation unit 41 Obtains the predetermined personal information given the ID identical to or corresponding to the personal information ID included in the transferred table. Similarly, the medical image information preparation unit 41 obtains the image information given the ID identical to or corresponding to the image information ID included in the transferred table. By combining the personal information and the image information, the medical image information preparation unit 41 prepares to reproduce the medical image information (step S32). The medical image information is transferred to the display unit 43 (step S33) and displayed in the display unit 43 (step S34).

If the user needs to use the image data for the purpose of researching images resulting from the image data, the image information including the image data can be utilized. Also, when a maintenance service staff needs to use the image data co refer to images based on the image data during the maintenance service, the image information can be utilized. For any other purpose, which does not directly relate to medical practices for the patient associated with the image data, the image information stored in the image information storage unit 31 can be utilized.

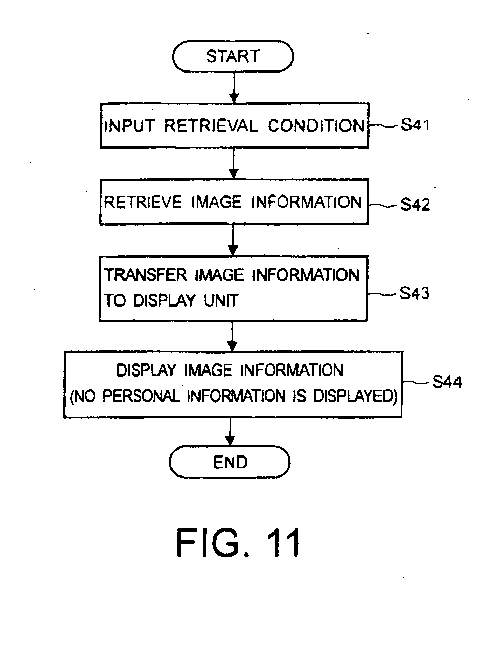

FIG. 11 is a flowchart showing an exemplary flow of outputting the image information in the medical image storage apparatus 10 according to the first embodiment. When the user, the maintenance service staff, or other person who needs to obtain images (hereinafter collectively referred to as an operator) desires to obtain the images, the operator inputs retrieval conditions from the operation unit 6 (step S41). The retrieval conditions may be one or more of a type of a medical imaging apparatus, an imaging condition, imaged part information, and the like. Selectable items corresponding to these retrieval conditions may be displayed in the display unit 43 in response to a predetermined operation through the operation unit 6. In this case, the operator may only need co select desired conditions with respect to the displayed items.

In response to the input or selection, the image information retrieval unit 42 retrieves the image information stored in the image information storage unit 31 based on the retrieval conditions (step S42). The a retrieved image information is transferred to the display unit 43 (step S43). The image information is displayed as images, a type of a medical imaging apparatus, an imaging condition, imaged part information, and the like while no personal information is displayed in the display unit 43 (step S44).

The personal information is stored separately from the image information and cannot be matched with the image information without decrypting the encrypted table. The encrypted table can be decrypted only with the decryption key. The decryption key is administered by the patient. Therefore, the personal informacion is secured from the operator when the images are required for the purpose which does not directly relate to medical practices for the patient associated with the images. On the other hand, when the user needs to display the medical image information including the personal information, the user can display the medical image information, using the decryption key administered by the patient. In other words, for the patient, the personal informacion included in the medical image intormation cannot be referred to even by the user without the patient's administering decryption key. This may mean that the personal information is referred to only in front of the patient.

In FIG. 1, the operation unit 6 includes the IC card reader 61. Accordingly, the user may need to borrow the IC card 81 from the patient and bring it co the medical image storage apparatus 10, which is typically provided where the patient is not permitted to enter. Otherwise, the patient may be asked to come to the medical image storage unit 10 and insert his/her IC card 81 into the IC card reader 61. These may not be practical. One possible application may be a use of the IC card reader 61 remotely connected to a main body of the medical image storage unit 10 through the hospital LAN or a direct communication line. The IC card reader 61 is still operative as part of the operation unit 6 while the IC card reader 81 may be provided in a consultation room where the patient is usually consulted by the user.

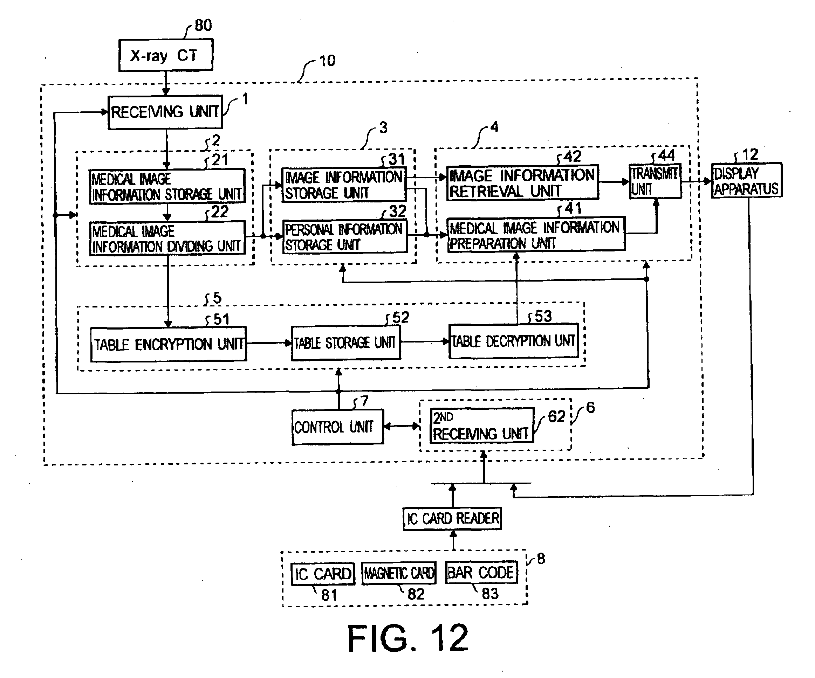

Another practical way of receiving information stored, for example, in the IC card 81 is shown in FIG. 12. The operation unit 6 includes a second receiving unit 62 instead of the IC card reader 61 shown in FIG. 1. The second receiving unit 62 is connected to an external IC card reader 11 through the hospital LAN. The external IC card reader 11 may be directly connected to the hospital LAN or through a medical image display apparatus such as, for example, a workstation. The external IC card reader 11 may be provided in the consultation room. The second receiving unit 62 receives the unique information, the encryption key, and/or the decryption key read from the IC card 81 by the external IC card reader 11.

Also in FIG. 12, the output unit 4 includes a transmitting unit 44 instead of the display unit 43 shown in FIG. 1. The medical image information prepared by the medical image information preparation unit 41 is transmitted to an external medical image display apparatus 12, for example, through the hospital LAN (omitted in FIG. 12). Similarly, the image information retrieved by the image information retrieval unit 42 is transmitted to the external medical image display apparatus 12. The external medical image display apparatus 12 may be the same apparatus as that connected to the external IC card reader 11 so that the external medical image display apparatus 12 can request the medical image information or the image information and receive the requested information.

The rest of the configuration of the medical image storage apparatus 10 may be similar to the corresponding units shown in FIG. 1 and therefore, the detail explanation is omitted herein.

### (Second Embodiment)

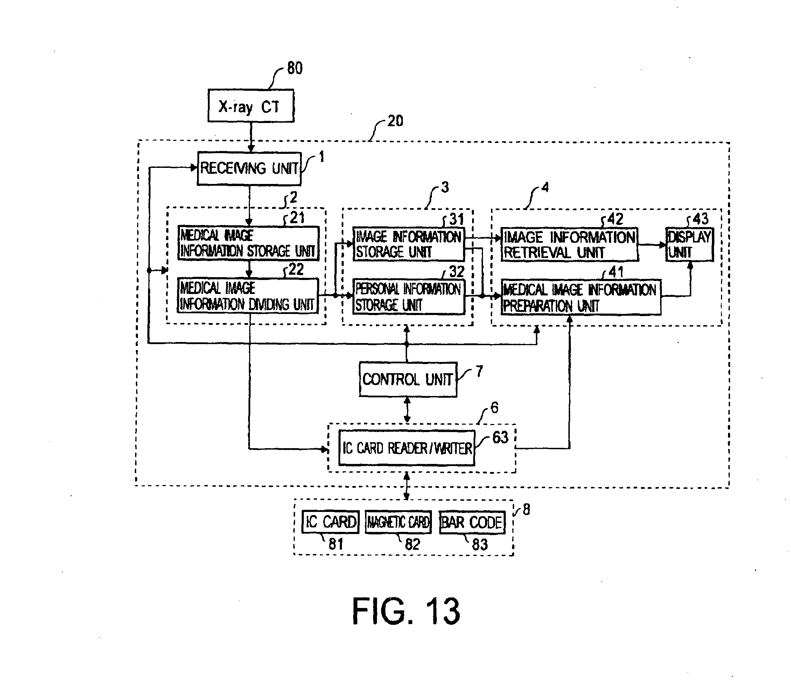

In the second embodiment, the table is not encrypted but still made unavailable to the user and the operator. FIG. 13 is a block diagram showing an exemplary configuration of a medical image storage apparatus according to the second embodiment. As shown in FIG. 13, a medical image storage apparatus 20 does not include the table processing unit. The operation unit 6 also does not include the IC card reader 61 but an IC card reader/writer 63. Ocher configuration of the medical image storage apparatus 20 may be similar to those included in the medical image storage apparatus 10 shown in PIG. 1. Therefore, the detail explanation of the similar configuration is omitted herein.

The IC card reader/writer 63 may read the unique informacion from the IC card 81. Further, the IC card reader/writer 63 writes the cable co the IC card 81 and reads the table from the IC card 81. The IC card reader/wricer 63 may correspond to the 'first unit configured to make the cable unavailable' and also to the 'second unit configured co recapture the table'.

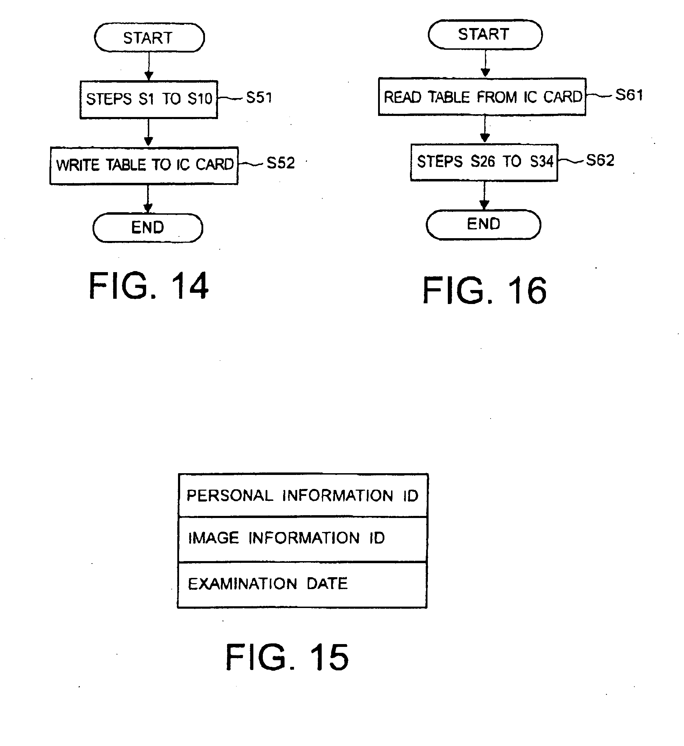

FIG. 14 is a flowchart showing an exemplary flow of writing the table to the IC card 81 according to the second embodiment The table is prepared in the medical image information divining unit 22 in accordance with steps S1 to S10 shown in FIG.4 (step S51). The table is transferred co the IC card reader/writer 63 and written to the IC card 81 (step S52). Therefore, the medical image information cannot be reproduced without the IC card 81, Since the table concerning a patient is stored in the IC card 81 kept and administered by the patient, the cable may not include the unique information as shown in FIG. 15.

FIG. 16 is a flowchart showing an exemplary flow of outputting the medical image information according to the second embodiment. The user may input an instruction to start obtaining the medical image information through the operation unit 6. In order to obtain and output the medical image information except for the time of the image interpretation which may have been completed before the encryption, the user then needs to ask the patient to insert the IC card 81 into the IC card reader/writer 63. The patient inserts the IC card 81 to the IC card reader/writer 63 and the IC card reader/writer 63 reads the table from the IC card 81 (step S61). It may not be necessary for the user to input the unique information of the patient. After the table read from the IC card 81 is transferred to the medical image information preparation unit 41, the medical image information may be displayed in the display unit 43 in accordance with steps S26 to S34 shown in FIG. 10 (step S62).

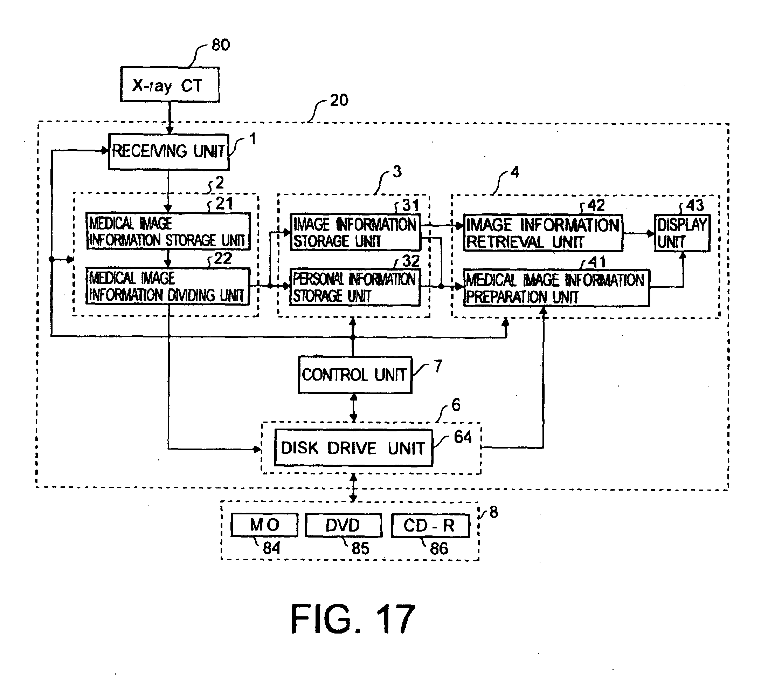

As a modification to the operation unit 6, the operation unit 6 may include a disk drive unit 64 as shown in FIG. 17. The disk drive unit 64 may correspond to the 'first unit' and the 'second unit'. FIG. 17 is a block diagram showing a modified exemplary configuration of the medical image storage apparatus according to the second embodiment. The disk drive unit 64 may write to and read from a magneto-optic disk (MO) 84, a digital versatile disk (DVD) 85, or a compact disk recordable (CD-R) 86. The MO 84, the DVD 85. and the CD-R 86 are other examples of the external memory medium 8 although applicable recordable disks are not limited to these examples.

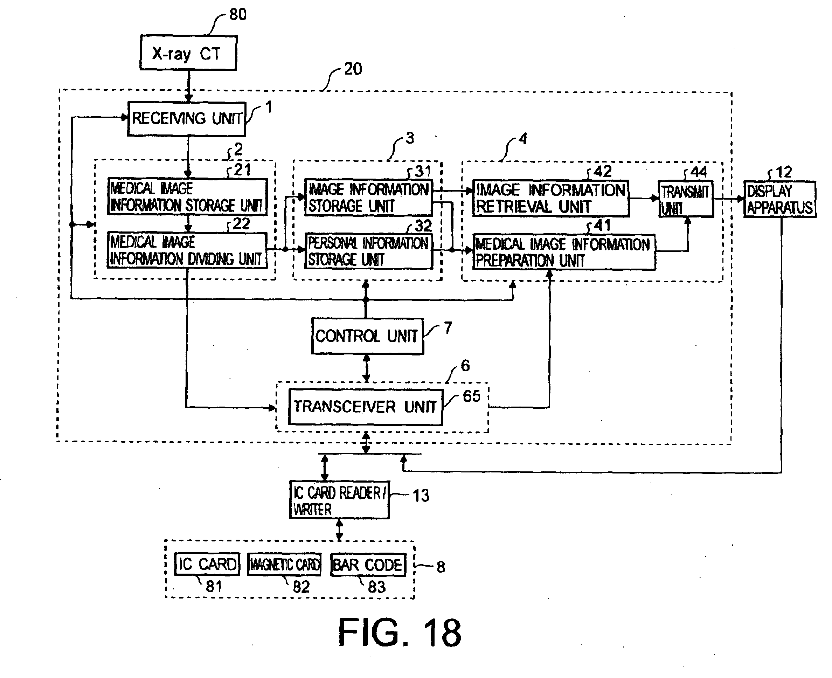

As shown in FIG. 18, the medical image storage apparatus 20 may not include the IC card reader/writer 63 but a transceiver unit 65. The transceiver unit 65 may correspond to the 'first unit' and the 'second unit', and is connected to an external IC card reader/writer 13 through the hospital LAN. The external IC card reader/writer 13 may be directly connected to the hospital LAN or through a medical image display apparatus such as, for example, a workstation. The external IC card reader/writer 13 may be provided in the consultation room. The transceiver unit 65 transmits the table transferred from the medical image information dividing unit 22 to the external IC card reader/writer 13. The transceiver unit 65 also receives the table read from the IC card 81 from the external IC card reader/writer 13.

Also in FIG. 18, the output unit 4 includes the transmitting unit 44 instead of the display unit. The medical image information prepared by the medical image information preparation unit 41 is transmitted to the external medical image display apparatus 12, for example, through the hospital LAN (omitted in FIG. 18). Similarly, the image information retrieved by the image information retrieval unit 42 is transmitted to the external medical image display apparatus 12. The external medical image display apparatus 12 may be the same apparatus as that connected to the external IC card reader/writer 13 so that the medical image display apparatus 12 can request the medical image information or the image information and receive the requested information.

### (Third Embodiment)

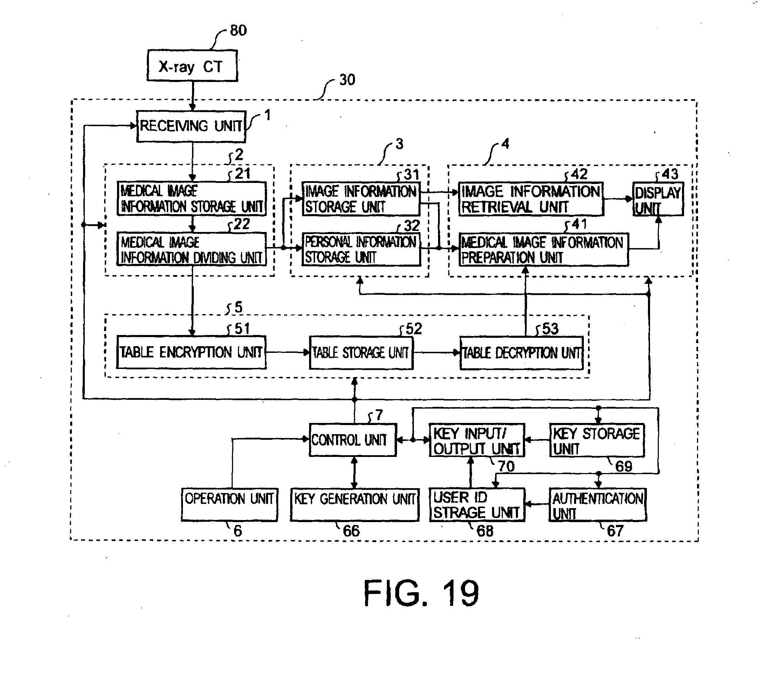

The unique information has been described for distinguishing the patient in the first embodiment. The unique information may alternatively be used to distinguish the user. FIG. 19 is a block diagram showing an exemplary configuration of a medical image storage apparatus according to the third embodiment. As shown in FIG. 19, the operation unit 6 may not include the IC card reader but a medical image storage apparatus 30 includes a key generation unit 66, an authentication unit 67, a user ID (ID: identification information) storage unit 68, a key storage unit 69, and a key input/output unit 70.

The key generation unit 66 generates an encryption key and a decryption key for each user. The encryption key and the decryption key may be the same one key. The authentication unit 67 authenticates the user who is allowed to login the medical image storage apparatus 30 by authentication information such as, for example, a user ID and a password input from the operation unit 6. In response to the authentication, the user logins the medical image storage apparatus 30. The user ID storage unit 68 stores the user ID authenticated by the authentication unit 67. That is, the user ID scored in the user ID storage unit 68 pertains to the user currently logging in the medical image storage apparatus 30. The key storage unit 69 stores the encryption key and the decryption key in relationship to the user ID with respect to one or more users. The key input/output unit 70 obtains the user ID scored in the user ID storage unit 68 and retrieves from the key storage unit 69 the encryption key or the decryption key which corresponds to the user ID. The key input/output unit 70 then outputs the encryption key or the decryption key to the control unit 7 so that the encryption key is transferred to the table encryption unit 51 when the encryption is required and the decryption key is transferred to the table decryption unit 53 when the decryption is required. The rest of the configuration of the medical image storage apparatus 30 may be similar to the corresponding units shown in FIG. 1 and therefore, the detail explanation is omitted herein.

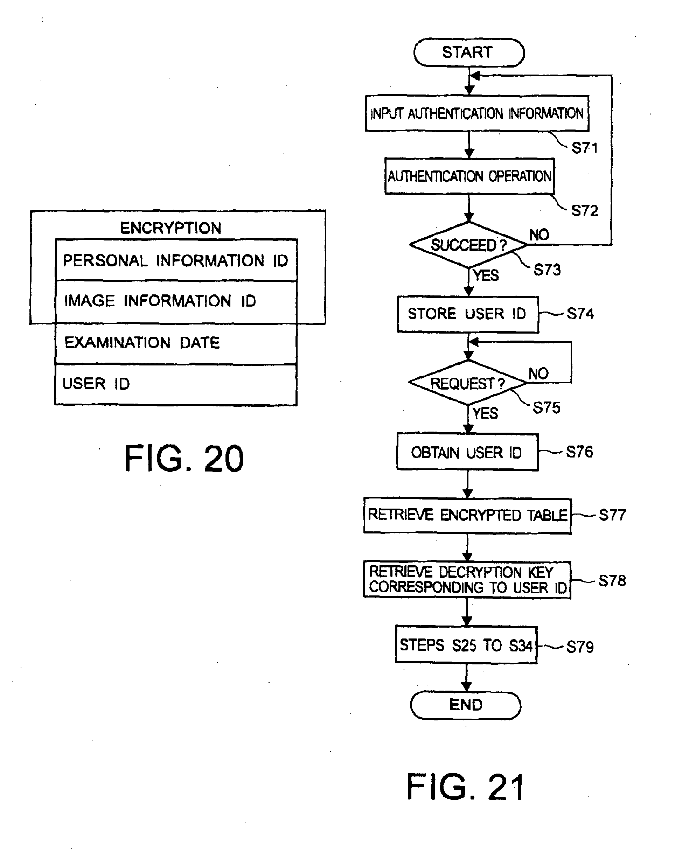

In this embodiment, the unique information included in the table is the user ID as shown in FIG. 20 and the user ID is not encrypted when the table is encrypted with the encryption key in the table encryption unit 51 as similar to FIG. 3B.

Outputting operations of the medical image information will be described with reference to a flowchart shown in FIG. 21. FIG. 21 is a flowchart showing an exemplary flow of outputting the medical image information in the medical image storage apparatus 30 according to the third embodiment.

The user who needs to obtain the medical image information with respect to his/her patient inputs the authentication information from the operation unit 6 (step S71). The authentication information is transferred to the authentication unit 67 and authenticated by the authentication unit 67 (step S72). If the authentication succeeds (step S73), the user ID of the user corresponding to or included in the authentication information is stored as the user ID of the current user in the user ID storage unit 68 (step S74). The user is allowed co login the medical image storage apparatus 30 and may start to use the medical image storage apparatus 30.

The user may make a request of obtaining the medical image information through the operation unit 6 (step S75). In response to the request, the control unit 7 obtains the user ID from the user ID storage unit 68 (seep S76) and transfers the user ID to the table decryption unit 53. The table decryption unit 53 retrieves one or more encrypted tables including user ID identical to the transferred user ID (step S77). The concrol unit 7 also controls the key input/output unit 70 so as co retrieve from the key storage unit 69 the decryption key corresponding to the user ID stored in the user ID storage unit 68 (step S78). After the retrieval, the decryption key is transferred co the cable decryption unit 53, and the medical image information is displayed in the display unit 43 in accordance with steps S25 to S34 shown in FIG. 10 (step S79).

According co the third embodiment, the user can obtain the medical image information with respect to only the user's patient. Therefore, although the patient's personal information included in the medical image information is not always referred in front of the patient, the patient may be secured that his/her medical image information is referred to only by the user or cannot be referred co at least without through the user's operation.

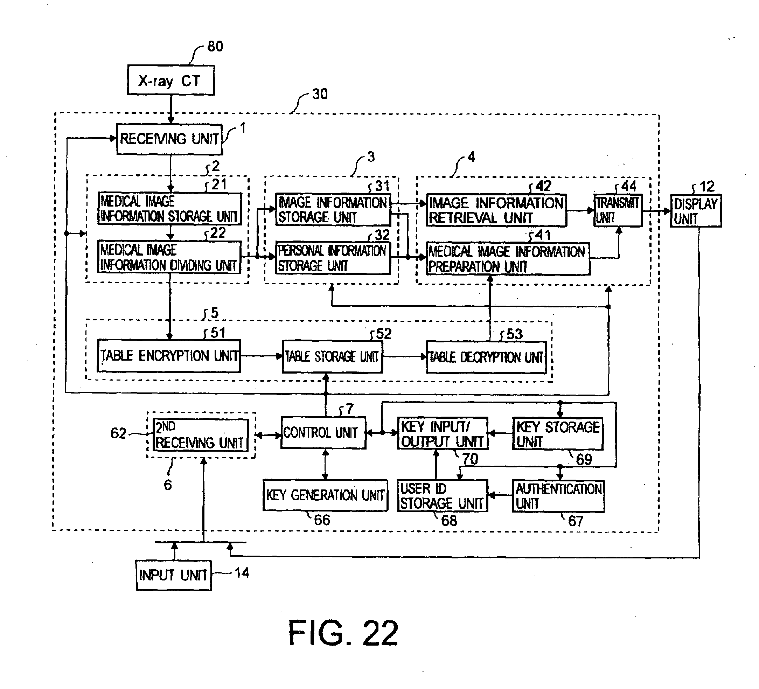

The input of the authentication information and the request may be made from an external input unit as shown in FIG. 22. The operation unit 6 includes the second receiving unit 62. The second receiving unit 62 is connected to an external input unit 14, for example, through the hospital LAN. The external input unit 14 may be connected to the hospital LAN directly or as part of a medical image display apparatus such as, for example, a workstation. The external input unit 14 may be provided in the consultation room. The second receiving unit 62 receives the authentication information and the request input from the external input unit 14.

Also in FIG. 22, the output unit 4 includes the transmitting unit 44 instead of the display unit shown in FIG. 19. The medical image information prepared by the medical image information preparation unit 41 is transmitted to the external medical image display apparatus 12, for example, through the hospital LAN (omitted in FIG. 22). Similarly, the image information retrieved by the image information retrieval unit 42 is transmitted to the external medical image display apparatus 12. The external medical image display apparatus 12 may be the same apparatus as that incorporating the external input unit 14 so that the medical image display apparatus 12 can request the medical image information or the image information and receive the requested information.

The rest of the configuration of the medical image storage apparatus 30 may be similar to the corresponding units shown in FIG. 19 and therefore, the detail explanation is omitted herein.

In the third embodiment, the user ID has been used as the unique information. Information distinguishing a consultation department provided in the hospital such as, for example, internal medicine or surgery may be used as an alternative example of the unique information.

### (Fourth Embodiment)

In the first to third embodiments, the medical image information has been received from the X-ray CT apparatus and divided into the personal information and the image information in the medical image information dividing unit. According co the fourth embodiment the personal information may be obtained separately from the medical image information so that it is not necessary to divide the medical image information.

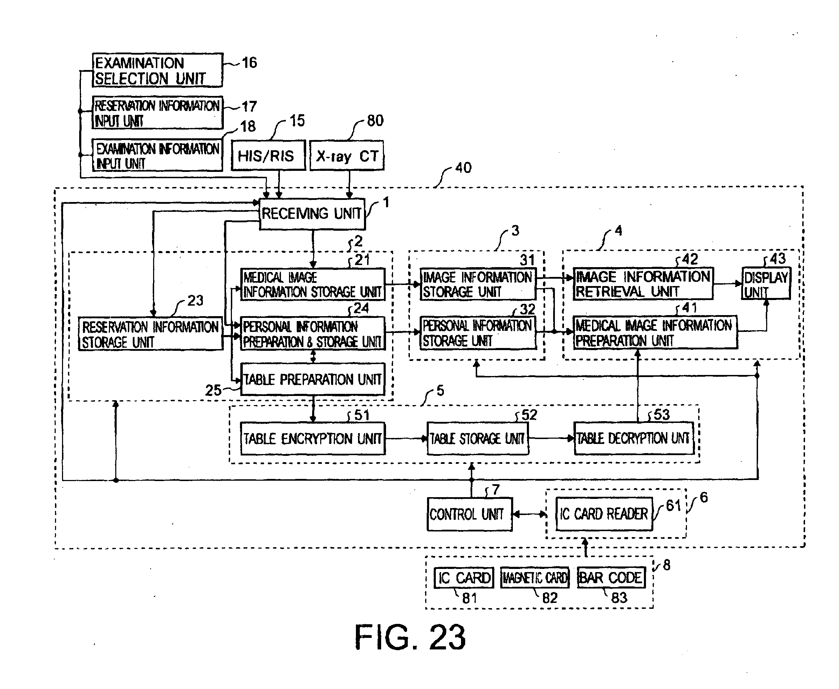

FIG. 23 is a block diagram showing an exemplary configuration of a medical image storage apparatus according to the fourth embodiment. As shown in FIG. 23, a medical image storage apparatus 40 may be connected to a hospital information system and/or a radiology information system (hereinafter referred to as a HIS/RIS) 15, for example, through the hospital LAN. The medical image storage apparatus 40 may also be connected to one or more of an examination selection unit 16, a reservation information input unit 17, and an examination information input unit 18. The examination selection unit 16, the reservation information input unit 17, and the examination information input unit 18 may be provided in one or more external apparatuses, which are connected co the receiving unit 1, for example, through the hospital LAN. The image processing unit 2 may not include the medical image information dividing unit but a reservation information storage unit 23, a personal information preparation and storage unit 24, and a table preparation unit 25 as well as the medical image information storage unit 21.

The receiving unit 1 receives the medical image information from the X-ray CT apparatus 80 as described in the first embodiment. The received medical image information is transferred to the medical image information storage unit 21. The medical image information storage unit 21 removes predetermined personal information included in the medical image information in accordance with a predetermined rule. Accordingly, the image information is prepared. Dummy information may be incorporated into the image information instead of the removed predetermined personal information.

The receiving unit 1 also receives examination reservation information from the HIS/RIS 15. The examination reservation information concerning a forthcoming imaging examination of the patient is typically input to the HIS/RIS 15 so as to reserve the imaging examination for the patient. The examination reservation information usually includes various types of information of a forthcoming examination and a patient, so may include personal information of the patient. The reservation information storage unit 23 stores the examination reservation information received by the receiving unit 1. The personal information preparation and storage unit 24 extracts and prepares predetermined personal information from the examination reservation information stored in the reservation information storage unit 23 in accordance with the predetermined rule. The prepared predetermined personal information i s stored in the personal information preparation and storage unit 24.

The receiving unit 1 may also receive examination selection information from the examination selection unit 16. The examination selection information may be transferred to the reservation information storage unit 23 so that the personal information preparation and storage unit 24 conducts the above operation with respect to the examination reservation information selected based on the examination selection information. The receiving unit 1 may further receive the examination reservation information from the reservation information input unit 17. The examination reservation information received from the reservation information input unit 17 may also be stored in the reservation information storage unit 23 and treated in a similar manner to those received from the HIS/RIS 15. Examination information received from the examination information input unit 18 may be transferred to the personal information preparation and storage unit 24. The personal informacion preparation and storage unit 24 may also or alcernacively extract the predetermined personal information from the examination information received from the examination information input unit 18 if necessary although the personal information preparation and storage unit 24 extracts the predetermined personal information from the examination reservation information as described above. One or more of the examination reservation information, the examination selection information, and the examination information may correspond to 'examination-relating information'.

The table preparation unit 25 issues the personal information ID and the image information ID. The personal information ID is given to the predetermined personal information stored in the personal information preparation and storage unit 24. The image information ID is given to the image information stored in the medical image information storage unit 21. The table preparation unit 25 also prepares a table of a relationship between the predetermined personal information and the image information in a manner similar to that described in the first embodiment. The table is transferred co the cable encryption unit 51.

The rest of the configuration of the medical image storage apparatus 40 may be similar to the corresponding units shown in FIG. 1 and therefore, the detail explanation is omitted herein.

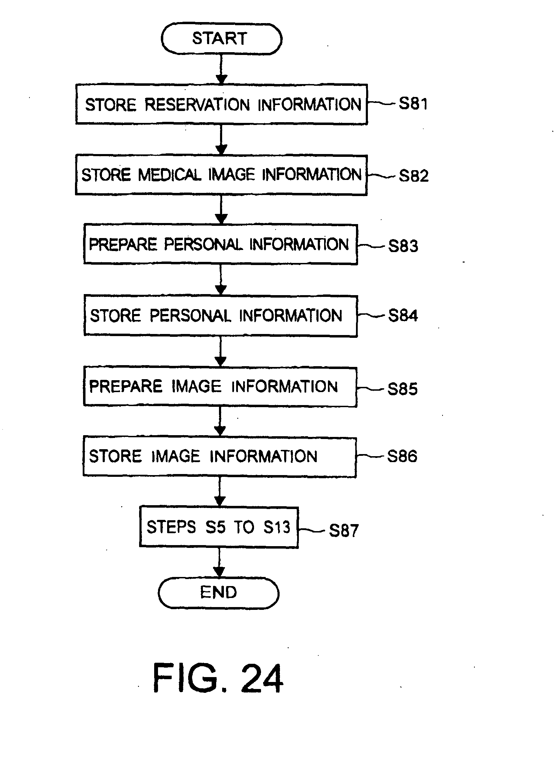

Storing operations of the predetermined personal information and the image information will be described with reference to a flowchart shown in FIG. 24. FIG. 24 is a flowchart showing an exemplary flow of storing the medical image information in the medical image storage apparatus 40 according to the fourth embodiment.

The storing operation may start similarly co step S1 in FIG. 4. However, the examination reservation information may be received from the HIS/RIS 15 by the receiving unit 1 in advance of receiving the medical image information from the X-ray CT apparatus 80. When the receiving unit 1 has received the examination reservation information from the HIS/RIS 15 and/or the reservation informacion input unit 17, the examination reservation information is transferred to the reservation information storage unit 23. The examination reservation information is stored in the reservation information storage unit 23, for example, until the a reserved examination is completed or the medical image information is received by the receiving unit 1 (step S81). Also, when the receiving unit 1 has received the medical image information from the X-ray CT apparatus 80, the medical image information is transferred to and stored in the medical image information storage unit 21 (step S82).

The examination reservation information is transferred to the personal information preparation and storage unit 24, for example, in response that the medical image information has been received by the receiving unit 1. When the receiving unit 1 has received the examination information from the examination information input unit 18. the examination information is also scored in the personal informacion preparation and storage unit 24. The personal information preparation and storage unit 24 extracts predetermined personal information, which is included in the examination reservation information and the examination information and is appropriate co be combined with the image information. The predetermined personal information may be prepared as a result of the extraction (step S83) and stored in the personal information preparation and storage unit 24 (step S84).

In the medical image information storage unit 21, the predetermined personal information is removed from the medical image information so that the image information is prepared (step S85). The prepared image information is stored in the medical image information storage unit 21 (step S86).

Although the table is prepared by the table preparation unit 25 and also the personal information ID and the image information ID are issued by the table preparation unit 25 as described above, the table is encrypted and the medical image information is made unavailable in a manner similar co those in steps S5 co S13 shown in FIG. 4 (step S87).

Outputting operations of the medical image information may be accomplished in a manner shown in FIG. 10.

### (Fifth Embodiment)

The patient's personal information may be secured that his/her medical image information is obtained only by limited one or more users or cannot be obtained at least without through these users' operation according to the fifth embodiment.

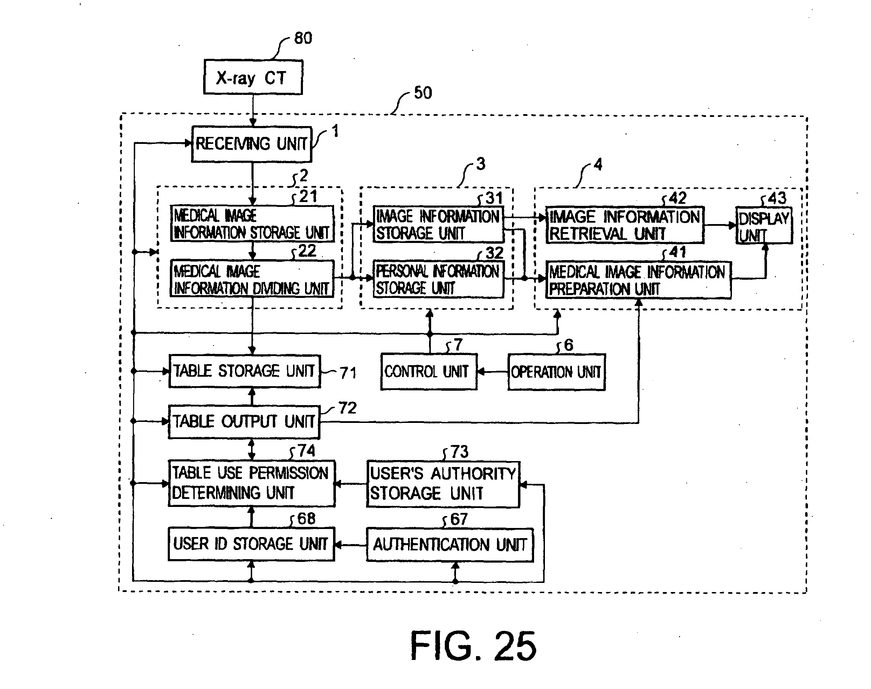

FIG. 25 is a block diagram showing an exemplary configuration of a medical image storage apparatus according to the fifth embodiment. A medical image storage apparatus 50 does not include the table processing unit but the authentication unit 67, the user ID storage unit 68, a table storage unit 71, a table output unit 72, a user's authority storage unit 73, and a table use permission determining unit 74.

The authentication unit 67 and the user ID storage unit 68 are operative in a manner as described in the third embodiment. The cable storage unit 71 stores the table prepared in the medical image information dividing unit 22. The table output unit 72 outputs the table stored in the table storage unit 71 to the medical image information preparation unit 41 in accordance with an instruction from the table use permission determining unit 74. The user's authority storage unit 73 stores the second table showing if the user is permitted co use the table. For example, the second table may show that users A and C (or user IDs A and C) are permitted to use the table but the user B (or user ID B) cannot with respect to a specific patient (or specific unique information). The second cable may include such information with respect to one or more patients. The user's authority storage unit 73 may alternatively store the second table showing with respect to which unique informacion the user ie permitted to use the cable. For example, the second table may show that a user A (or user ID A) is permitted to use the table concerning patients A, B, and C (or unique information A, B, and C) while a user B (or user ID B) is permitted to use the table concerning the patients A and B (or the unique information A and B). And the second cable may also show that a user C (or user ID C) is not permitted to use any table or may not include information of the user C (or the user ID C), which means the user C is not a permitted user of any cable. The table use permission determining unit 74 determines whether or not the user who logged in the medical image storage unit 50 is permitted co use one or more tables based on the second table stored in the user's authority storage unit 73.

The rest of the configuration of the medical image storage apparatus 50 may be similar to the corresponding units shown in FIG. 1 and therefore, the detail explanation is omitted herein.

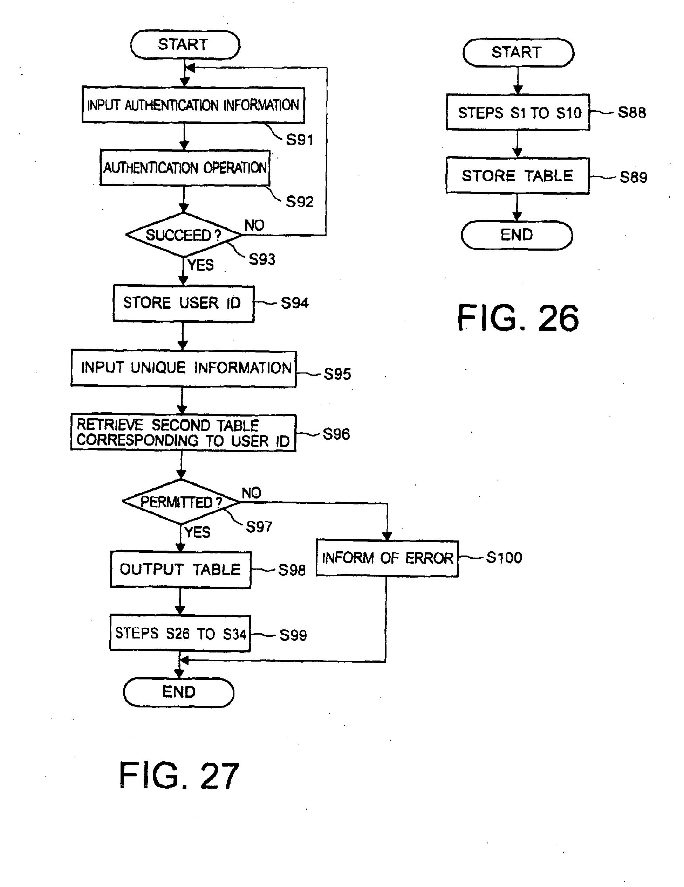

FIG. 26 is a flowchart showing an exemplary flow of scoring the medical image information in the medical image storage apparatus 50 according to the fifth embodiment. In storing operations of the medical image information, the table may be prepared in accordance with steps S1 to S10 shown in FIG. 4 (step S88). The table is stored in the cable storage unit 71 (step S89). This table is made unavailable by the user's authority storage unit 73, the cable use permission determining unit 74, and the table output unit 72. In other words. the user's authority storage unit 73, the table use permission determining unit 74, and the table output unit 72 may collectively correspond co the 'first unit' and the 'second unit'.

Outputting operations of the medical image information will be described with reference to a flowchart shown in FIG. 27. FIG. 27 is a flowchart showing an exemplary flow of outputting the medical image information in the medical image storage apparatus 50 according to the fifth embodiment.

The user who needs to obtain the medical image information with respect to a specific patient inputs the authentication information from the operation unit 6 (step S91). The authentication information is transferred to and authenticated by the authentication unit 67 (step 392). If the authentication succeeds (step S93), the user ID of the user corresponding co or included in the authentication information is stored as the user ID of the current user in the user ID storage unit 68 (step S94). The user is allowed to login the medical image storage apparatus 50 and may start to use the medical image storage apparatus 50.

The user may input unique information concerning the specific patient from the operation unit 6 (step S95). The unique information may be transferred to the table use permission determining unit 74. The table use permission determining unit 74 obtains the current user ID from the user ID storage unit 68 and, for example, retrieves information of the second table concerning the obtained user ID from the user's authority storage unit 73 (step S96). The table use permission determining unit 74 then determines whether the current user ID is permitted to use table(s) of the transferred unique information or not (step S97). When the table use permission determining unit 74 determines that the current user ID is permitted to use the table(s) of the transferred unique information, the table output unit 72 outputs one or more cables of the unique information to the medical image information preparation unit 41 (step S98). The medical image information may be displayed in accordance with steps S26 to S34 shown in FIG. 10 (step S99). If the table use permission determining unit 74 does not determines that the current user ID is permitted co use the table(s) of the transferred unique information in step S97, the control unit 7 controls the output unit 4 so that the display unit 43 displays information of an error that the user is not permitted to obtain the medical image information of the patient concerning the input unique information (step S100).

### (Sixth Embodiment)

The feature of the medical image storage apparatus described in the first to fifth embodiments may also be implemented in a medical imaging apparatus such as one mentioned in the first embodiment. Two examples will be described below although any other one or more embodiments described above or their combination may also be applied to the medical imaging apparatus.

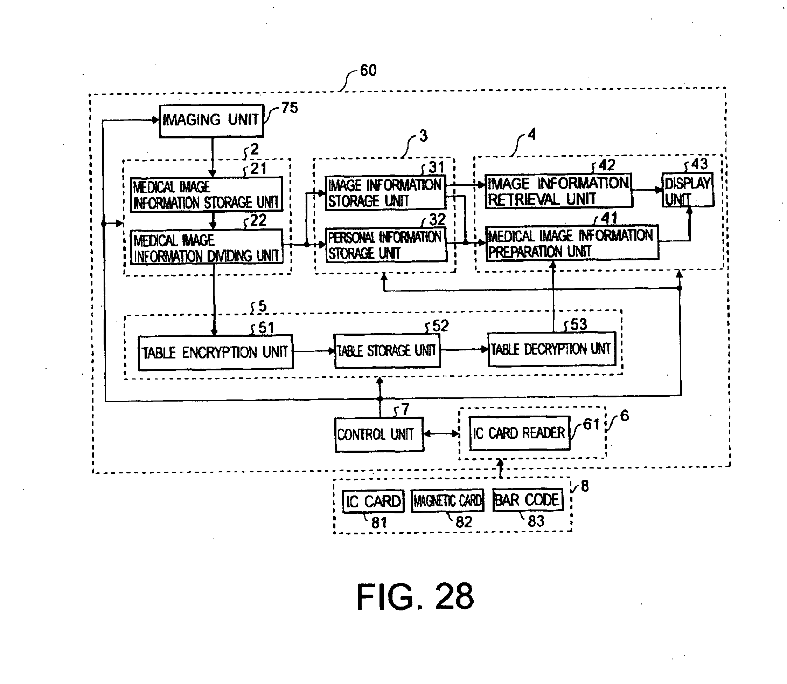

FIG. 28 is a block diagram showing an exemplary configuration of a medical imaging apparatus according to the sixth embodiment. A medical imaging apparatus 60 may include an imaging unit 75, the image processing unit 2, the storage unit 3, the output unit 4, the table processing unit 5, the operation unit 6, and the control unit 7. The external memory medium 8 may be used for the operation unit 6.

The imaging unit 75 generates medical image information and may include, for example, an X-ray tube, an X-ray detector, a data acquisition system, a reconstruction section, an information preparation section, and the like when the medical imaging apparatus 60 is an X-ray CT apparatus. The X-ray tube generates x-ray towards a patient and the X-ray detector detects X-ray information resulting from the generated x-ray. The detected X-ray informacion is acquired by the data a acquisition system and is reconstructed to produce image data by the reconstruction section. The information preparation section prepares medical image information by combining the image data and its accompanying information. The accompanying information may be obtained from the operation unit 6 or from an external apparatus (not shown in FIG. 28), for example, through the hospital LAN. The medical image information is transferred co the image processing unit 2.

The image processing unit 2, the storage unit 3, the output unit 4, the table processing unit 5, the operation unit 6, and the control unit 7 may be operative in a manner similar co the corresponding units in FIG. 1, and therefore, the detail explanation is omitted herein.

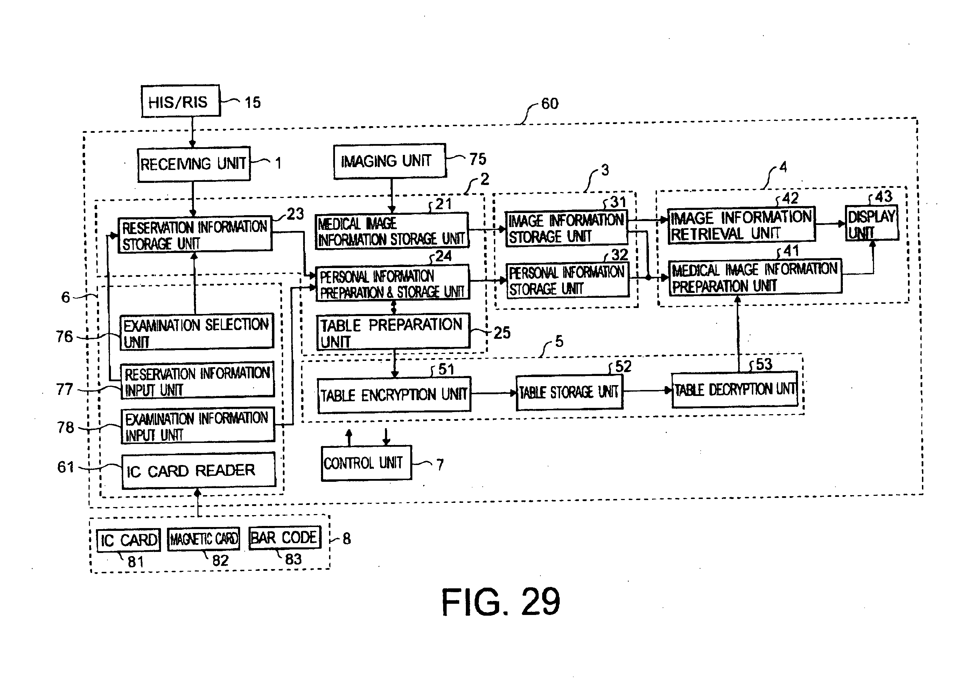

A modification of the medical imaging unit 60 will be described wich reference to FIG. 29. PIG. 29 is a block diagram showing a modified exemplary configuration of the medical imaging apparatus 60 according to the sixth embodiment. As shown in FIG. 29, the medical imaging apparatus 60 include the receiving unit 1, which may be connected co the HIS/RIS 15, for example, through the hospital LAN. The image processing unit 2 may not include the medical image information dividing unit but includes the reservation information storage unit 23, the personal information preparation and storage unit 24, and the table preparation unit 25 as well as the medical image information storage unit 21.

The receiving unit 1 receives examination reservation information from the HIS/RIS 15 and the received examination reservation information is stored in the reservation information storage unit 23 as described in the fourth embodiment. Also as described in the fourth embodiment, the personal information preparation and storage unit 24 extracts, prepares, and stores predetermined personal information from the examination reservation information stored in the reservation information storage unit 23. The table preparation unit 25 is operative in a manner similar to that described in the fourth embodiment.

The operation unit 6 includes an examination selection unit 76, a reservation information input unit 77, and an examination information input unit 78 as well as the IC card reader 61. The examination selection unit 76 may be used to select an examination from a listed plurality of reserved examinations so that the medical imaging apparatus 60 conducts imaging with respect to the selected examination. The personal information preparation and storage unit 24 may conduct the described operation with respect co the examination reservation information concerning the selected examination. The reservation information input unit 77 is provided to input the examination reservation information to be stored in the reservation information storage unit 23. but may not be used so often. The examination information input unit 78 may be used to input the examination information. The examination information input from the examination information input unit 78 is transferred to the personal information preparation and storage unit 24. The personal information preparation and storage unit 24 may also or alternatively extract the predetermined personal information from the transferred examination information if necessary although the personal information preparation and storage unit 24 extracts the predetermined personal information from the examination reservation information as described above.

The imaging unit 75 is operative as described with reference to FIG. 28. The medical image information generated from the imaging unit 75, however, may not include personal information. Therefore, the medical image information storage unit 21 stores the medical image information as the image information.

The rest of the configuration of the medical imaging apparatus 60 may be similar to the corresponding units shown in FIG. 28 and therefore, the detail explanation is omitted herein. In FIG. 29, the connection is omitted between the control unit 7 and other units.

### (seventh Embodiment)

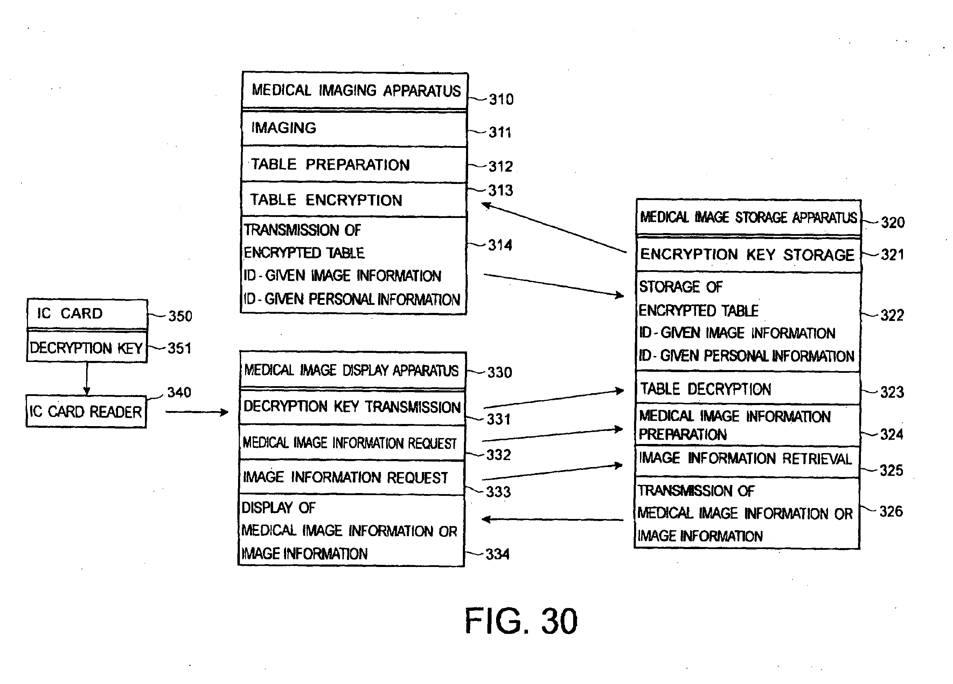

FIG. 30 is an illustration showing an example of a medical image system according to the seventh embodiment. The medical image system may include a medical imaging apparatus 310, a medical image storage apparatus 320. and a medical image display apparatus 330, which may be connected to one another through a hospital LAN. The medical image display apparatus 330 may be connected to an IC card reader 340. The IC card reader 340 reads information stored in an IC card 350.

The medical imaging apparatus 310 may be any type of medical imaging apparatus mentioned in the first embodiment. The medical imaging apparatus 310 may have features of, but which are not limited to, imaging 311. table preparation 312, table encryption 313, and transmission of encrypted table, ID-given image information, and ID-given predetermined personal information 314. The imaging feature 311 may be implemented in a unit such as, for example, the imaging unit 75. The cable preparation feature 312 may be implemented in a unit such as, for example, the medical image information dividing unit 22. The table encryption feature 313 may be implemented in a unit such as, for example, the table encryption unit 51. The transmission feature 314 may be implemented in a unit such as, for example, a unit like the transmitting unit 44 if the transmitting unit 44 is provided in the medical imaging apparatus 60 in the sixth embodiment.

The medical image storage apparatus 320 may be a filing server and have features of, but which are not limited to, encryption key storage 321, storage of the encrypted table, the ID-given image information, and the ID-given personal information 322, table decryption 323, medical image information preparation 324, image information retrieval 325, and transmission of the medical image information or the image information 326. The encryption key storage feature 321 may be implemented in a unit such as, for example, the control unit 7. The storage feature 322 may be implemented in units such as, for example, the storage unit 3 and the table storage unit 52. The table decryption feature 323 may be implemented in a unit such as, for example, the table decryption unit 53. The medical image information preparation feature 324 may be implemented in a unit such as, for example, the medical image information preparation unit 41. The image information retrieval feature 325 may be implemented in a unit such as, for example, the image information retrieval unit 42. The transmission feature 326 may be implemented in a unit such as, for example, the transmitting unit 44.

The medical image display apparatus 330 may be a workstation and used, for example, to interpret images in an image interpretation room, to observe images in a consultation room, or to research images in a conference room. The medical image display unit 330 may have features of, which are not limited to, decryption key transmission 331, medical image information request 332, image information request 333, and display of the medical image information or the image information 334. The decryption key transmission feature 331 may be implemented, for example, in a transceiver unit. The medical image information request feature 332 and the image information request feature 333 may be implemented, for example, in an input unit and the transceiver unit. The display feature 334 may be implemented, for example, in a display unit.

The IC card 350 may include a decryption key 351 corresponding to the encryption key. When the encryption key stored in the medical image storage apparatus 320 is a public key of a patient, the decryption key 351 is a private key corresponding to the public key. The IC card 350 may be kept and administered by the patient.

A communication flow among the medical imaging apparatus 310, the medical image storage apparatus 320, the medical image display apparatus 330, the IC card reader 340, and the IC card 350 will be described briefly below. When the medical image information is generated by the imaging feature 311, predetermined personal information and image information are prepared based on the medical image information, and also a table of a relationship between the predetermined personal information and the image information is prepared by the table preparation feature 312. The predetermined personal information and the image information are given IDs, respectively. The given IDs are included in the table. The table is encrypted with the encryption key by the table encryption feature 313. The encryption key may be obtained from the medical image storage apparatus 320. The encrypted table is transmitted to the medical image storage apparatus 320 by the transmission feature 314. Also, the ID-given personal information and the ID-given image information are transmitted to the medical image storage apparatus 320 by the transmission feature 314.

The transmitted encrypted table, the ID-given personal information, and the ID-given image information are received and stored in the medical image storage apparatus 320 by the storage feature 322.

When a user desires to obtain the medical image information of the patient, the user requests the medical image information and the request is transmitted from the medical image display apparatus 330 co the medical image storage apparatus 320 by the medical image information request feature 332. The user also asks the patient to insert his/her IC card 350 into the IC card reader 340. In response to the insertion, the IC card reader 340 reads the decryption key 351 from the IC card 350. The decryption key 350 is provided co the medical image display apparatus 330 and transmitted to the medical image storage apparatus 320 by the decryption key transmission feature 331. The medical image storage apparatus 320 decrypts the encrypted table with the decryption key 351 by the cable decryption feature 323. Based on the decrypted table, the medical image information is prepared and reproduced by the medical image information preparation feature 324. The medical image information is transmitted to the medical image display appatatus 330 by the transmission feature 326. In che medical image display apparatus 330, the medical image information is displayed by the display feature 334.

When the operator requests the image information by the image information request feature 333, the request is transmitted from the medical image display apparatus 330 to the medical image storage apparatus 320 by the image information request feature 333. In the medical image storage apparatus 320, the image information is retrieved by the image information retrieval feature 325. The image information is transmitted to the medical image display apparatus 330 by the transmission feature 326. In the medical image display apparatus 330, the image information is displayed by the display feature 334.

As described above, the encryption and the decryption may not always be implemented in the same apparatus. Also the image request and display may not be implemented in the apparatus which encrypts and/or decrypts the table. The features described in one or more of the embodiments described above may be implemented in different apparatuses and accomplished in totality within a predetermined medical image system. The seventh embodiment described with reference to FIG. 30 is only one example of such a system.

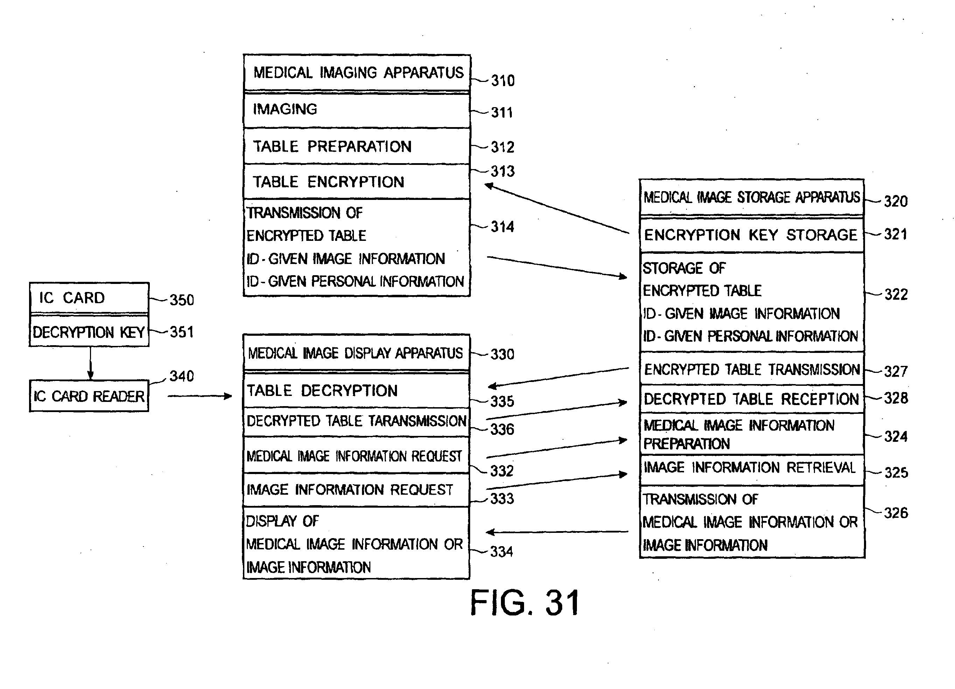

Further, the medical image system may a system as shown in FIG. 31. FIG. 31 is an illustration showing another example of the medical image system according to the seventh embodiment. As shown in FIG. 31, the medical image storage apparatus 320 may include an encrypted table transmission feature 327 and a decrypted table reception feature 328 instead of the table decryption feature 323. The medical image display apparatus 330 may include a table decryption feature 335 and a decrypted table transmission feature 336 instead of the decryption key transmission feature 331.

In response to the request of the medical image information by the medical image information request feature 332 in the medical image display apparatus 330, the encrypted table is transmitted from the medical image storage apparatus 320 by the encrypted table transmission feature 327. The decryption key 350 read by the IC card reader 340 is provided to the medical image display apparatus 330 and the encrypted table is decrypted with the decryption key by the table decryption feature 335. The decrypted cable is transmitted to the medical image storage apparatus 330 by the decrypted table transmission feature 336. The decrypted table is received by the decrypted table reception feature 328. Based on the decrypted table, the medical image information is prepared and reproduced by the medical image information preparation feature 324. The medical image information is transmitted to the medical image display apparatus 330 by the transmission feature 326. In the medical image display apparatus 330, the medical image information is displayed by the display feature 334.

When the decrypted table is transmitted from the medical image display apparatus 330 to the medical image storage apparatus 320, the decrypted cable may be transmitted through a network secured by, for example, SSL (Secure Socket Layer) or IP Sec.

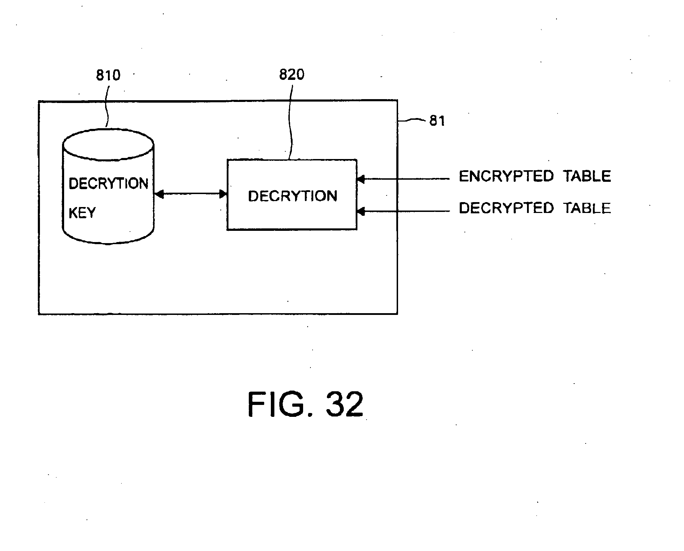

In the above embodiments in which the IC card 81 is used, the IC card 81 may be operative as part of one of the apparatuses 10 to 60, and 330 and include a memory 810 and a decryption unit (or feature) 820, as shown in FIG. 32. The memory 810 stores the decryption key. The IC card 81 may receive the encrypted table and the received table is decrypted by the decryption unit 820. The decrypted table is provided co the one apparatus. According to this example, the decryption key itself is not transmitted through a network so as to be secured. The decrypted table may be sent to the apparatus through a network secured by, for example, SSL (Secure Socket Layer) or IP Sec..

Also one or more of the first co seventh embodiments may be combined as another embodiment.

The embodiments of the present invention described above are examples described only for making it easier to understand the present invention, and are not described for the limitation of the present invention. Consequently, each component and element disclosed in the embodiments of the present invention may be redesigned or modified to its equivalent within a scope of the present invention. Furthermore, any possible combination of such components and elements may be included in a scope of the present invention as long as an advantage similar to those obtained according to the above disclosure in the embodiments of the present invention is obtained.

Numerous modifications and variations of the present invention are possible in light of the above teachings. It is therefore to be understood that within the scope of the appended claims, the invention may be practiced otherwise than as specifically described herein.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. A medical image storage apparatus, comprising:
a receiving unit configured to receive medical image information resulting from a medical imaging apparatus;
a table preparation unit configured to prepare a table of a relationship between predetermined personal information concerning the medical image information and image information prepared by removing the predetermined personal information from the medical image information;
a first storage unit configured to store the predetermined personal information;
a second storage unit configured to store the image information;
a first unit configured co make the table unavailable;
a second unit configured to recapture the table; and
an output unit configured co read the predetermined personal information from the first storage unit and the image information from the second storage unit based on the recaptured table and co prepare and output the medical image information.

2. The apparatus according to claim 1, wherein the first unit is an encryption unit configured to encrypt the table and the second unit is a decryption unit configured to decrypt the encrypted table.

3. The apparatus according to claim 2, further comprising a second receiving unit configured to receive a private key of a patient.
Wherein the encryption unit is further configured to store a public key corresponding to the private key and encrypts the table with the public key: and the decryption unit decrypts the encrypted table with the private key.

4. The apparatus according to claim 3, wherein the private key is administered by the patient.

5. The apparatus according to claim 3, wherein the second receiving unit receives the private key read from an external memory medium by an external information reading unit through a network.

6. The apparatus according to claim 3, wherein the second receiving unit is an information reading unit which reads the private key stored in an external memory medium.

7. The apparatus according co claim 6. wherein the external memory medium is an IC card.

8. The apparatus according to claim 2, further comprising a second receiving unit configured to receive a encryption key and a decryption key,
wherein: the encryption unit encrypts the table with the encryption key; and the decryption unit decrypts the encrypted cable with the decryption key.

9. The apparatus according to claim 8, wherein the encryption key and the decryption key are administered by a patient.

10. The apparatus according to claim 8, wherein the second receiving unit receives the encryption key and the decryption key read from an external memory medium by an external information reading unit through a network.

11. The apparatus according to claim 8, wherein the second receiving unit is an information reading unit which reads the encryption key and the decryption key stored in an external memory medium.

12. The apparatus according to claim 1, wherein:
the first storage unit stores the predetermined personal information with first identification information, the second storage unit stores the image information with second identification information; and the table includes the first identification information and the second identification information.

13. The apparatus according co claim 12. wherein the first unit is an encryption unit configured to encrypt the table and the second unit is a decryption unit configured to decrypt the encrypted table.

14. The apparatus according to claim 12, wherein the table is combined with examination date information concerning the medical image information and unique information.

15. The apparatus according to claim 14, further comprising a second receiving unit configured co receive second unique information of a patient scored in an external memory medium, wherein the second unit is further configured to compare the second unique information to the unique information and recaptures the table when the second unique information and the unique information have a predetermined correspondence.

16. The apparatus according co claim 14, further comprising a second receiving unit configured to receive second unique information of a user of the apparatus, wherein the second unit is further configured to compare the second unique information to the unique information and recaptures the table when the second unique information and the unique information have a predetermined correspondence.

17. The apparatus according to claim 14, further comprising a second receiving unit configured to receive second unique in formation of a consultation department, wherein the second unit is further configured to compare the second unique information to the unique information and recaptures the cable when the second unique information and the unique information have a predetermined correspondence.

18. The apparatus according co claim 1, wherein the first unit is an information writing unit configured to write the table co a external memory medium and the second unit is an information reading unit configured co read the table from the external memory medium.

19. The apparatus according to claim 18, wherein:
the external memory medium is an IC card; the information writing unit is an IC card writer; and the information reading unit is an IC card reader.

20. The apparatus according to claim 18, wherein:
the external memory medium is a memory disk; and the information writing unit and the information reading unit are operative as a disk drive unit.

21. The apparatus according to claim 1, wherein:
the first unit transmits the table to an external information writing unit which writes the table to an external memory medium; and the second unit receives the cable from an external information reading unit which reads the table from the external memory medium.

22. The apparatus according to claim 1, wherein the table preparation unit is further configured to divide the medical image information into the predetermined personal information and the image information.

23. The apparatus according to claim 1, wherein the receiving unit is further configured to receive examination-relating information with respect to imaging by the medical imaging apparatus and to prepare the predetermined personal information based on the examination-relating information.

24. The apparatus according to claim 23, wherein at least part of the examination-relating information is received from an external information system through a network.

25. The apparatus according to claim 1, wherein the first unit and the second unit are operative as a table use permission determining unit configured to include a second table showing if a user of the apparatus is permitted to use the table.

26. The apparatus according to claim 1, wherein the first unit and the second unit are operative as a table use permission determining unit configured to include a second table showing with respect to which unique information a user of the apparatus is permitted to use the table, the unique information being combined to the cable.

27. The apparatus according to claim 1, wherein the image information includes dummy information instead of the predetermined personal information.

28. The apparatus according to claim 1, wherein the first unit is rendered inoperative for a predetermined period after imaging by the medical imaging apparatus.

29. The apparatus according co claim 1, wherein the output unit is further configured to output the image information stored in the first storage unit independently of recapturing the cable in the second unit.

30. The apparatus according co claim 29, wherein:
the image informacion includes image data and condition informacion of at least one of a type of the medical imaging apparatus, an imaging condition, and an imaged part of the patient; and the output unit is further configured to retrieve the image information based on the condition information and to output the image data.

31. A medical imaging apparatus, comprising:
an imaging unit configured to generate medical image information;
a table preparation unit configured to prepare a table of a relationship between predetermined personal information concerning the medical image information and image information based on the medical image information;
a first storage unit configured to store the predetermined personal information;
a second storage unit configured to store the image information;
a first unit configured co make the table unavailable;
a second unit configured to recapture the table; and
an output unit configured to read the predetermined personal information from the first storage unit and the image information from the second storage unit based on the recaptured table and to prepare and output the medical image information.

32. A medical image system including a medical imaging apparatus and a medical image storage apparatus, the system comprising:
an imaging unit configured to generate medical image information;
a table preparation unit configured to prepare a table of a relationship between predetermined personal information concerning the medical image information and image information based on the medical image information;
a first storage unit configured to store the predetermined personal information:
a second storage unit configured to store the image information;
a first unit configured to make the table unavailable;
a second unit configured to recapture the table; and
an output unit configured to read the predetermined personal information from the first storage unit and the image information from the second storage unit based on the recaptured table and to prepare and output the medical image information.

33. A method of storing medical image information, comprising:
receiving medical image information resulting from a medical imaging apparatus;
preparing a table of a relationship between predetermined personal information concerning the medical image information and image information prepared by removing the predetermined personal information from the medical image information;
storing the predetermined personal information;
storing the image information; and
making the table unavailable.

34. The method according to claim 33. further comprising:
recapturing the table;
reading the predetermined personal information from the first storage unit and the image information from the second storage unit based on the recaptured table;
preparing the medical image information: and
outputting the medical image information.

35. A method of scoring medical image information, comprising:
generating medical image information;
preparing a table of a relationship between predetermined personal information concerning the medical image information and image information based on the medical image information;
storing the predetermined personal information:
storing the image information; and
making the table unavailable.

36. The method according to claim 35, further comprising:
recapturing the table;
reading the predetermined personal information from the first storage unit and the image information from the second storage unit based on the recaptured table;
preparing the medical image information; and
outputting the medical image information.

37. The apparatus according co claim 1, wherein the first unit is an encryption unit configured to encrypt the table and the second unit is an IC card including a decryption unit configured to decrypt the encrypted table.
